(19) Europäisches Patentamt
European Patent Office
Office européen des brevets

(11) EP 1 928 551 B1

(12) EUROPEAN PATENT SPECIFICATION

(45) Date of publication and mention of the grant of the patent:
**30.05.2012 Bulletin 2012/22**

(21) Application number: **06773127.3**

(22) Date of filing: **12.06.2006**

(51) Int Cl.:
***A61P 13/04*** (2006.01) ***A61P 13/12*** (2006.01)
***A61P 1/18*** (2006.01) ***A61K 38/44*** (2006.01)

(86) International application number:
**PCT/US2006/023115**

(87) International publication number:
**WO 2006/135925 (21.12.2006 Gazette 2006/51)**

(54) **METHODS TO REDUCE OXALATE CONCENTRATION BY ADMINISTRATION OF OXALATE OXIDASE CRYSTALS**

VERFAHREN ZUR REDUZIERUNG DER OXALAT-KONZENTRATION DURCH VERABREICHUNG VON OXALAT-OXIDASE-KRISTALLEN

PROCEDE DE REDUCTION DE LA TENEUR EN OXALATE PAR ADMINISTRATION DE CRISTAUX D'OXALATE OXYDASE

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.06.2005 US 689468 P**

(43) Date of publication of application:
**11.06.2008 Bulletin 2008/24**

(60) Divisional application:
**11178385.8**

(73) Proprietor: **Althea Technologies, Inc.**
**San Diego, CA 92121 (US)**

(72) Inventors:
- **MARGOLIN, Alexey, L.**
  **Newton, Massachusetts 02161 (US)**
- **YANG, Mark, X.**
  **Newton, MA 02458 (US)**
- **MCGRATH, Margaret, Ellen**
  **Somerville, MA 02145 (US)**
- **SHENOY, Bhami, C.**
  **South Grafton, Massachusetts 01560 (US)**

(74) Representative: **Graf von Stosch, Andreas**
**Graf von Stosch**
**Patentanwaltsgesellschaft mbH**
**Prinzregentenstrasse 22**
**80538 München (DE)**

(56) References cited:
**WO-A-2004/060920 US-A- 6 011 001**

- **RAMAKRISHNAN V ET AL: "INVESTIGATION WITH CHITOSAN-OXALATE OXIDASE-CATALASE CONJUGATE FOR DEGRADING OXALATE FROM HYPEROXALURIC RAT CHYME" INDIAN JOURNAL OF BIOCHEMISTRY AND BIOPHYSICS, COUNCIL OF SCIENTIFIC & INDUSTRIAL RESEARCH, NEW DEHLI, IN, vol. 34, no. 4, August 1997 (1997-08), pages 373-378, XP008065786 ISSN: 0301-1208**
- **SVEDRUZIC D ET AL: "The enzymes of oxalate metabolism: unexpected structures and mechanisms" ARCHIVES OF BIOCHEMISTRY AND BIOPHYSICS, NEW YORK, US, US, vol. 433, no. 1, 1 January 2005 (2005-01-01), pages 176-192, XP004665470 ISSN: 0003-9861**
- **PUNDIR C S ET AL: "ISOLATION, PURIFICATION, IMMOBILIZATION OF OXALATE OXIDASE AND ITS CLINICAL APPLICATIONS" HINDUSTAN ANTIBIOTICS BULLETIN, vol. 35, no. 1/2, February 1993 (1993-02), pages 173-182, XP002044102 ISSN: 0018-1935**
- **WOO E-J ET AL: "Barley oxalate oxidase is a hexameric protein related to seed storage proteins: evidence from X-ray crystallography" FEBS LETTERS, ELSEVIER, AMSTERDAM, NL, vol. 437, no. 1-2, 16 October 1998 (1998-10-16), pages 87-90, XP004258496 ISSN: 0014-5793**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 928 551 B1

**Description**

## BACKGROUND OF THE INVENTION

Oxalic acid is a dicarboxylic acid of the formula $HO_2C-CO_2H$. Oxalic acid exists primarily as oxalate in biological organisms, which is the salt form of oxalic acid. Oxalate is found in foods, such as, *e.g.*, spinach, rhubarb, strawberries, cranberries, nuts, cocoa, chocolate, peanut butter, sorghum, and tea. Oxalate is also a metabolic end-product in humans and other mammals. It is excreted by the kidneys into the urine. When combined with calcium, oxalic acid produces an insoluble product, calcium oxalate, which is the most prevalent chemical compound found in kidney stones.

Because mammals do not synthesize enzymes that degrade oxalate, oxalate levels in an individual are normally held in check by excretion and low absorption of dietary oxalate. Elevated concentrations of oxalate are associated with a variety of pathologies, such as primary hyperoxaluria, enteric hyperoxaluria, and idiopathic hyperoxaluria. Leumann et al., Nephrol. Dial. Transplant. 14:2556-2558 (1999) and Earnest, Adv. Internal Medicine 24:407-427 (1979). Increased oxalate can be caused by consuming too much oxalate from foods, by hyperabsorption of oxalate from the intestinal tract, and by abnormalities of oxalate production. Hyperabsorption of oxalate in the colon and small intestine can be associated with intestinal diseases, including hyperabsorption caused by diseases of bile acid or fat malabsorption, ileal resection, or, for example, by steatorrhea due to celiac disease, exocrine pancreatic insufficiency, intestinal disease, or liver disease.

Hyperoxaluria, or increased urinary oxalate excretion, is associated with a number of health problems related to the deposit of calcium oxalate in the kidney tissue (nephrocalcinosis) or urinary tract (*e.g.*, kidney stones, urolithiasis, and nephrolithiasis). Calcium oxalate may also be deposited in, *e.g.*, the eyes, blood vessels, joints, bones, muscles, heart and other major organs, causing damage to the same. *See, e.g.,* Leumann et al., J. Am. Soc. Nephrol. 12:1986-1993 (2001) and Monico et al., Kidney International 62:392-400 (2002). The effects of increased oxalate levels can appear in a variety of tissues. For example, deposits in small blood vessels cause painful skin ulcers that do not heal, deposits in bone marrow cause anemia, deposits in bone tissue cause fractures or affect growth in children, and calcium oxalate deposits in the heart cause abnormalities of heart rhythm or poor heart function.

Existing methods to treat elevated oxalate levels are not always effective and intensive dialysis and organ transplantation may be required in many patients with primary hyperoxaluria. Existing therapies for various hyperoxalurias include high-dose pyridoxine, orthophosphate, magnesium, iron, aluminum, potassium citrate, cholestyramine, and glycosaminoglycan treatment, as well as regimes for adjusting diet and fluid intake, for dialysis, and for surgical intervention, such as renal and liver transplantation. These therapies (*e.g.*, low-oxalate or low-fat diet, pyridoxine, adequate calcium, and increased fluids), are only partially effective and they may have undesirable adverse side effects, such as the gastrointestinal effects of orthophosphate, magnesium, or cholesyramine supplementation and the risks of dialysis and surgery. Accordingly, methods that safely remove oxalate from the body are needed. Moreover, methods that degrade oxalate to reduce oxalate levels in a biological sample are advantageous over a therapy, for example, that solely blocks absorption or increases clearance of oxalate.

The use of oxalate degrading bacteria to reduce oxalate in an individual is referred to, for example, in U.S. Patent Nos. 6,200,562, 6,355,242, and 6,699,469. U.S. Patent Pub. No. 2004/0234514, on the other hand, refers to the administration of enzymes that are involved in oxalate pathways. However, these oxalate degrading enzymes are sensitive to the harsh acid environment of the stomach. The '514 publication refers to the use of enteric coatings, for example, to overcome these stability problems. Nevertheless, highly active, stable, and otherwise advantageous forms of an oxalate degrading enzyme, such as oxalate oxidase, are needed to treat oxalate-related disorders.

As current therapies are not optimal, there is also a need for methods and compositions to treat or prevent disorders associated with elevated oxalate concentrations in an individual, such as in individuals with oxalate-related disorders. As one example, methods to treat primary hyperoxaluria are also needed, to allow individuals to delay or avoid surgery such as organ transplantation.

## SUMMARY OF THE INVENTION

This invention provides cross-linked crystals of oxalate oxidase for use in therapeutic methods, for example, to allow treatment of an oxalate-related disorder by oral administration of the cross-linked crystalline oxalate oxidase. The cross-linked crystals of oxalate oxidase can also, for example, be used in methods that are effective to control oxalate concentrations or to minimize damage caused by calcium oxalate deposits in an individual.
In particular embodiments, the cross-linking agent is multifunctional, and in certain embodiments, the agent is a bifunctional agent, such as glutaraldehyde.

In some instances, the crystals comprise oxalate oxidase from a natural source, such as plants, bacteria and fungi, in particular from wheat, barley, maize, oat, rice, spinach, sorghum, banana, and rye. In other instances the oxalate oxidase is recombinantly produced.

The present invention also provides a medical use for reducing oxalate concentration in a mammal, comprising administering cross-linked oxalate oxidase crystals to the mammal. The medical uses result in a reduction of oxalate concentration by at least 10%, at least 20%, at least 30%, or at least 40%.

The cross-linked crystals are administered orally. In other instances, the crystals are administered via the upper gastrointestinal tract.

The invention also provides a medical use to treat, prevent, or slow the progression of a disorder associated with elevated oxalate concentration in a mammal, comprising administering a cross-linked oxalate oxidase crystal to the mammal. In particular embodiments, the disorder is selected from a kidney disorder, bone disorder, liver disorder, gastrointestinal disorder, and pancreatic disorder. In further aspects, the disorder is selected from primary hyperoxaluria, enteric hyperoxaluria, idiopathic hyperoxaluria, ethylene glycol poisoning, urolithiasis and nephrolithiasis.

. The foregoing summary and the following description are not restrictive of the invention as claimed.

## BRIEF DESCRIPTION OF THE DRAWINGS

Figure 1 shows the apparent mobility of purified oxalate oxidase ("OXO") (soluble and crystalline forms) in a 4-20% SDS PAGE gradient gel under reducing (lane 1-4) and non-reducing conditions (lane 6-8). Under reducing conditions, OXO is a monomer. Under non-reducing conditions, the majority of OXO is present as a hexamer. Lane 1: MW marker; lane 2: soluble OXO; lane 4: crystalline OXO; lane 6: soluble OXO; lane 8: crystalline OXO; lane 10: molecular weight markers. Crystalline OXO was prepared according to the batch crystallization of Example 6.

Figure 2 shows Periodic Acid Schiff (PAS) staining for glycopeptides and Coomassie blue staining ("Normal") of duplicate samples resolved in a 4-20% tris-glycine gel that was cut into two halves. The left half (lanes 1-5) was stained with Coomassie blue and the right half (lanes 7-10) was stained with PAS. Lane 1: MW marker; lane 3: soluble OXO; lane 5: crystalline OXO; lane 7: soluble OXO; lane 9: crystalline OXO. Crystalline OXO was prepared according to the batch crystallization of Example 6.

Figure 3 shows oxalate oxidase reactivity of OXO separated in a non-reducing SDS gel. In-situ oxalate oxidase analysis detected activity in various OXO preparations. The activity was associated with a polypeptide having an apparent molecular weight of approximately 120 kDa. Lane 1: Molecular weight Marker; lane 3: purified soluble OXO; lane 5: desalted soluble OXO in 10 mM MES pH 6.0; lane 7: crystalline OXO (crystallized from 40% (v/v) PEG 400, MES pH 6.0, 5% (w/v) PEG 3000) dissolved in water; lane 9: crystalline OXO (crystallized from 40% (v/v) PEG 600, CHES pH 9.5) dissolved in water.

Figure 4 is a plot comparing the kinetic profiles of soluble OXO and cross-linked OXO crystals (see Example 6), showing that the crystallized OXO retains at least 95% of the activity of uncrystalized OXO.

Figures 5A and 5B are a series of photographs of OXO crystals grown by vapor diffusion.

Figure 6 is a photograph(s) of OXO crystals grown by the microbatch method.

Figure 7 depicts OXO crystals grown by the batch method (Figure 7A), and of cross-linked OXO crystals (Figure 7B) grown by the batch method.

Figure 8 depicts the results of oxalate oxidase (OXO) therapy in a mouse model for primary hyperoxaluria. AGT1 knock-out male mice were administered soluble OXO via gavage (Figure 8A), cross-linked OXO crystals (Figure 8B), or a mock treatment (control group). Urinary oxalate and creatinine were measured in 24 hour urine samples. Error bars represent the standard error (SE) (n=5-8), which is calculated by dividing the standard deviation by the square root of n. An asterisk represents a statistically significant ($P<0.05$) difference between the control and experimental groups.

Figure 9 shows the results of OXO therapy in a rat model for enteric hyperoxaluria. Sprague Dawley male rats were administered cross-linked OXO crystals (15 mg/rat) or a mock treatment (control group). Urinary oxalate and creatinine were measured in 24 hour urine samples. Error bars represent the SE (n=6). An asterisk represents a statistically significant ($P<0.05$) difference between the control and experimental groups.

Figure 10 shows sequence information for SEQ ID NOS:1-3.

Figure 11 depicts a graph of the oxalate oxidase activity (%) of oxalate oxidase crystals cross-linked with glutaraldehyde (4%) as compared to the oxalate oxidase activity of soluble oxalate oxidase at various pHs.

Figure 12 shows reduction in urinary oxalate levels from controls during 1-11 days of oral OXO-CLEC treatment of EG AGT1 KO mice. Treatment groups (n=5) received OXO-CLEC orally at the dose 50, (adequate amount of enzyme slurry was mixed with 5gm food, freeze dried and each morning food containers were re-filled with ~7gm of food/enzyme mixture). Match control group had n=3 mice and was given same type of food without test article. Each bar represents mean value ± SE.

Figure 13 shows reduction in urinary oxalate levels from controls after 31 days of oral OXO-CLEC treatment of EG AGT1 KO. Treatment groups (n=11) received OXO-CLEC orally at the dose 50, (adequate amount of enzyme slurry was mixed with 3.5gm food, freeze dried and each morning food containers were re-filled with ~7gm of food/enzyme mixture). Match control group had n=11 mice and was given same type of food without test article. Basal urine oxalate levels are shown for day -3. Each bar represents mean value ± SE. *Indicates significant difference between

control group and treatment group. The results are analyzed by unpaired two tail Student's t-test. At the beginning of the study each group had n=11 mice, but several mice died during the course of the study due to ethylene glycol challenge; Presented are only mice that were alive at the particular day of urine oxalate measurements at the end of the study CONT group n=2 and 50 mg OXO-CLEC group n=7. Therefore, statistical sigilificance is less relevant. Rather, these results coupled with those described below provide evidence of the effectiveness of the treatments according to this invention.

Figure 14 shows the efficacy of oral OXO-CLEC treatment on maintaining the normal kidney function in extreme hyperoxaluria conditions measured by creatinine clearance. Shown are only mice that survived the entire one month study period; 50 mg group (n=7) and CONT group (n=2) mice. When compared with the control group, creatinine clearance was significantly higher in mice that received 50 mg of OXO-CLEC/ mouse/day. Each bar represents the mean value ± SE. Creatinine clearance in mice with normal kidney function is >10ml/h.

Figure 15 shows Yasue-positive calcium oxalate crystals in the kidney parenchyma of mice from treatment 50 mg OXO-CLEC group (Figure 15A) and control group (Figure 15C). The representative slides are shown at a magnification of 20 X. Normal kidney parenchyma with no calcium oxalate deposits shown in (A), moderate nephrocalcinosis shown in slide (B) and severe nephrocalcinosis shown in slide (C). The black arrows indicate calcium oxalate deposits, orange arrow indicates glomerulus with slightly changed morphology and brown arrow indicates large area with interstitial fibrosis.

Figute 16 shows Kaplan-Meyer survival curve that compares the survival times of ethylene glycol challenged mice that were treated with OXO-CLEC and those in the control group

## DETAILED DESCRIPTION

The present invention is based, in part, on the discovery and demonstration that administering crystals of oxalate oxidase (OXO) can treat hyperoxaluria. As described herein, crystalline oxalate oxidase administered orally or directly to the stomach can reduce oxalate levels in an individual, including an individual who is not consuming oxalate in their diet. Methods of administration of OXO crystals to treat various oxalate-related disorders are described herein. Additionally, OXO crystals and cross-linked crystals (CLECs) are provided, as are compositions comprising and using the same.

### Definitions

In order that the present invention may be more readily understood, certain terms are first defined. Additional definitions are set forth throughout the detailed description.

As used herein, a "**biological sample**" is biological material collected from cells, tissues, organs, or organisms, for example, to detect an analyte. Exemplary biological samples include a fluid, cell, or tissue sample. Biological fluids include, for example, serum, blood, plasma, saliva, urine, or sweat. Cell or tissue samples include biopsy, tissue, cell suspension, or other specimens and samples, such as clinical samples.

A "**crystal**" is one form of the solid state of matter, comprising atoms arranged in a pattern that repeats periodically in three dimensions *(see, e.g.,* Barret, Structure of Methals, 2nd ed., McGraw-Hill, New York (1952). A crystal form of a polypeptide, for example, is distinct from a second form—the amorphous solid state. Crystals display characteristic features including shape, lattice structure, percent solvent, and optical properties, such as, *e.g.*, refractive index.

An "**extracorporeal device**" is a structure that is not within the body for bringing a body fluid in contact with OXO crystals in the treatment of an individual. Preferably, an extracorporeal device is a device used for dialysis, including kidney dialysis, a device for continuous arteriovenous hemofiltration, an extracorporeal membrane oxygenator, or other device used to filter waste products from the bloodstream. Similarly, components of devices to filter waste products are encompassed by the term, including a tube, a porous material, or a membrane, for example. In particular, an extracorporeal device may be a dialysis device. It may also be a membrane of a dialysis device.

A "**functional fragment**" of OXO is a portion of an OXO polypeptide that retains one or more biological activities of OXO, such as the ability to catalyze the oxidation of oxalate. As used herein, a functional fragment may comprise terminal truncations from one or both termini, unless otherwise specified. For example, a functional fragment may have 1, 2,4, 5, 6, 8,10,12,15, or 20 or more residues omitted from the amino and/or carboxyl terminus of an OXO polypeptide. Preferably, the truncations are not more than 20 amino acids from one or both termini. A functional fragment may optionally be linked to one or more heterologous sequences.

The term "**individual**" refers to any mammal, including any animal classified as such, including humans, non-human primates, primates, baboons, chimpanzees, monkeys, rodents (*e.g.*, mice, rats), rabbits, cats, dogs, horses, cows, sheep, goats, pigs, etc.

The term "**isolated**" refers to a molecule that is substantially free of its natural environment. For instance, an isolated protein is substantially free of cellular material or other proteins from the cell or tissue source from which it is derived. The term refers to preparations where the isolated protein is sufficiently pure to be administered as a therapeutic

composition, or at least 70% to 80% (w/w) pure, more preferably, at least 80%-90% (w/w) pure, even more preferably, 90-95% pure; and, most preferably, at least 95%, 96%, 97%, 98%, 99%, 99.5%, 99.8% or 100% (w/w) pure.

As used herein, "**oxalate-related disorder**" refers to a disease or disorder associated with pathologic levels of oxalic acid or oxalate, including, but not limited to hyperoxaluria, primarily hyperoxaluria, enteric hyperoxaluria, idiopathic hyperoxaluria, ethylene glycol (oxalate) poisoning, idiopathic urinary stone disease, renal failure (including progressive, chronic, or end-stage renal failure), steatorrhoea, malabsorption, ileal disease, vulvodynia, cardiac conductance disorders, inflammatory bowel disease, cystic fibrosis, exocrine pancreatic insufficiency, Crohn's disease, ulcerative colitis, nephrocalcinosis, urolithiasis, and nephrolithiasis. Such conditions and disorders may optionally be acute or chronic. Oxalate-related disorders associated with kidneys, bone, liver, gastrointestinal tract, and pancreas are well known. Further, it is well known that calcium oxalate can deposit in a wide variety of tissues including, but not limited to the eyes, blood vessels, joints, bones, muscles, heart and other major organs leading to a number of oxalate-related disorders.

"**Oxalic acid**" exists predominantly in its salt form, oxalate (as salts of the corresponding conjugate base), at the pH of urine and intestinal fluid ($pk_{a1}$ = 1.23, $pK_{a2}$=4.19). Earnest, Adv. Internal Medicine 24:407-427 (1979). The terms "oxalic acid" and "oxalate" are used interchangeably throughout this disclosure. Oxalate salts comprising lithium, sodium, potassium, and iron (II) are soluble, but calcium oxalate is very poorly soluble in water, dissolving only to 0.58 mg/100 ml at 18 °C. Earnest, Adv. Internal Medicine 24:407-427 (1979). Oxalic acid from food is also referred to as dietary oxalate. Oxalate that is produced by metabolic processes is referred to as endogenous oxalate. Circulating oxalate is the oxalate present in a circulating body fluid, such as blood.

The terms "**therapeutically effective dose**," or "**therapeutically effective amount**," refer to that amount of a compound that results in prevention, delay of onset of symptoms, or amelioration of symptoms of an oxalate-related condition, including hyperoxaluria, such as primary hyperoxaluria or enteric hyperoxaluria. A therapeutically effective amount will, for example, be sufficient to treat, prevent, reduce the severity, delay the onset, or reduce the risk of occurrence of one or more symptoms of a disorder associated with elevated oxalate concentrations. The effective amount can be determined by methods well known in the art and as described in subsequent sections of this description.

The terms "**treatment," "therapeutic method**," and their cognates refer to treatment and prophylactic/preventative measures. Those in need of treatment may include individuals already having a particular medical disorder as well as those who may ultimately acquire the disorder. The need for treatment is assessed, for example, by the presence of one or more risk factors associated with the development of a disorder, the presence or progression of a disorder, or likely receptiveness to treatment of a subject having the disorder. Treatment may include slowing or reversing the progression of a disorder.

**Oxalate Oxidase**

As used herein, oxalate oxidase (OXO) refers to an oxalate:oxygen oxidoreductase enzyme. Oxalate oxidases are a group of well defined enzymes capable of catalyzing the molecular oxygen ($O_2$)-dependent oxidation of oxalate to carbon dioxide and hydrogen peroxide according to the following reaction.

$$HO_2C\text{-}CO_2H + O2 \rightarrow 2\ CO_2 + H_2O_2$$

Isoforms of oxalate oxidase, and glycoforms of those isoforms, are included within this definition. OXO from plants, bacteria and fungi are encompassed by the term, including the true cereal OXOs, such as wheat, barley, maize, oat, rice, and rye. Optionally, the OXO will additionally be capable of superoxide dismutase activity, such as barley OXO. In certain circumstances, OXO is a soluble hexameric protein, including a trimer of OXO glycoprotein dimers.

Oxalate oxidases are produced by higher plants, bacteria, and fungi and have oxalate:oxygen oxidoreductase enzymatic activity. Oxalate oxidases include those produced by the true cereals, such as wheat, barley, maize, oat, rice, and rye. These are generally identified as germin-type OXOs (G-OXOs), because wheat oxalate oxidase is also known as germin. The germin-like proteins (GLPs) are a large class of proteins sharing certain structural features. Other sources of OXO are moss, beet, spinach, sorghum, and banana. OXOs, such as G-OXOs, are active as, for example, hexameric glycoproteins. Some OXOs have also been reported to have superoxide dismutase activity.

Oxalate oxidases used to prepare the crystals and which are used in methods described herein may be isolated, for example, from a natural source, or may be derived from a natural source. As used herein, the term "derived from" means having an amino acid or nucleic acid sequence that naturally occurs in the source. For example, oxalate oxidase derived from barley will comprise a primary sequence of a barley oxalate oxidase protein, or will be encoded by a nucleic acid comprising a sequence found in barley that encodes an oxalate oxidase or a degenerate thereof. A protein or nucleic acid derived from a source encompasses molecules that are isolated from the source, recombinantly produced, and/or chemically synthesized or modified. The crystals provided herein may be formed from polypeptides comprising amino acid sequences of OXO, or a functional fragment of OXO that retains oxalate oxidizing activity. Preferably, the OXO

retains at least one functional characteristic of a naturally occurring OXO in addition to catalysis of the oxidation of oxalate, such as multimerization, manganese requirement, and/or superoxide dismutase activity.

## Isolated Oxalate Oxidase

Oxalate oxidases have been previously isolated and are thus available from many sources, including barley seedlings, roots, and leaves, beet stems, beet leaves, wheat germ, sorghum leaves, and banana peel. OXO may also be purchased from commercial purveyors, such as, *e.g.*, Sigma. Methods to isolate OXO from a natural source are previously described, for example, in the following references: Liu et al., Zhi Wu Sheng Li Yu Fen Zi Sheng Wu Xue Xue Bao 30:393-8 (2004) (Engl. Abst. at PMID 15627687); Rodriguiez-Lopez et al., FEBS Lett. 9:44-48 (2001); Pundir et al., Chin. J. Biotechnol. 15:129-138 (1999); and Aguilar et al., Arch. Biochem. Biophys. 366:275-82 (1999). These isolated oxalate oxidases may be used to form the crystals, cross-linked crystals and compositions of this invention. These crystals, cross-linked crystals, and compositions can then be used in the methods described herein.

## Recombinant Oxalate Oxidase

Alternatively, recombinant OXOs may be used to form the crystals and methods provided herein. In some instances, recombinant OXOs encompass or are encoded by sequences from a naturally occurring OXO sequence. Further, OXOs comprising an amino acid sequence that is homologous or substantially identical to a naturally occurring sequence are herein described. Also, OXOs encoded by a nucleic acid that is homologous or substantially identical to a naturally occurring OXO-encoding nucleic acid are provided and may be crystallized and/or administered as described herein.

Polypeptides referred to herein as "recombinant" are polypeptides which have been produced by recombinant DNA methodology, including those that are generated by procedures which rely upon a method of artificial recombination, such as the polymerase chain reaction (PCR) and/or cloning into a vector using restriction enzymes. "Recombinant" polypeptides are also polypeptides having altered expression, such as a naturally occurring polypeptide with recombinantly modified expression in a cell, such as a host cell.

In one embodiment, OXO is recombinantly produced from a nucleic acid that is homologous to a barley OXO nucleic acid sequence, and that is modified, e.g., to increase or optimize recombinant production in a heterologous host. An example of such a modified sequence is provided in SEQ ID NO:1 (nucleic acid), in which the nucleic acid sequence of the open reading frame of barley OXO is modified to reduce its GC content, and linked to an $\alpha$ Mating Factor secretion signal sequence and engineered restriction endonuclease cleavage sites. The amino acid sequence encoded by SEQ ID NO:1 is provided as SEQ ID NO:2. In an alternative iteration, OXO is recombinantly produced from SEQ ID NO:3, the unmodified barley nucleic acid sequence that is available at GenBank Accession No. L15737.

OXO polypeptides useful for forming OXO crystals may be expressed in a host cell, such as a host cell comprising a nucleic acid construct that includes a coding sequence for an OXO polypeptide or a functional fragment thereof. A suitable host cell for expression of OXO may be a yeast, bacteria, fungus, insect, plant, or mammalian cell, for example, or transgenic plants, transgenic animals or a cell-free system. Preferably, a host cell is capable of glycosylating the OXO polypeptide, capable of disulfide linkages, capable of secreting the OXO, and/or capable of supporting multimerization of OXO polypeptides. Preferred host cells include, but are not limited to *Pichia pastoris, Hansenula polymorpha, Saccharomyces cerevisiae, Schizosaccharomyces pombe, E. coli* (including *E. coli* Origami B), *Bacillus subtilis, Aspergillus,* Sf9 cells, Chinese hamster ovary (CHO), 293 cells (human embryonic kidney), and other human cells. Also transgenic plants, transgenic animals including pig, cow, goat, horse, chicken, and rabbit are suitable hosts for production of OXO.

For recombinant production of OXO, a host or host cell should comprise a construct in the form of a plasmid, vector, phagemid, or transcription or expression cassette that comprises at least one nucleic acid encoding an OXO or a functional fragment thereof. A variety of constructs are available, including constructs which are maintained in single copy or multiple copy, or which become integrated into the host cell chromosome. Many recombinant expression systems, components, and reagents for recombinant expression are commercially available, for example from Invitrogen Corporation (Carlsbad, CA); U.S. Biological (Swampscott, MA); BD Biosciences Pharmingen (San Diego, CA); Novagen (Madison, WI); Stratagene (La Jolla, CA); Deutsche Sammlung von Mikroorganismen und Zellkulturen GmbH (DSMZ), (Braunschweig, Germany). Altemativel, the recombinant OXO can be produced by the well known gene activation technology.

Recombinant expression of OXO is optionally controlled by a heterologous promoter, including a constitutive and/or inducible promoter. Promoters such as, e.g., the alcohol oxidase (AOX) promoter, the dihydroxyacetone synthase (DAS) promoters, the Gal 1,10 promoter, the phosphoglycerate kinase promoter, the glyceraldehyde-3-phosphate dehydrogenase promoter, alcohol dehydrogenase promoter, copper metallothionein (CUP1) promoter, acid phosphatase promoter, and T7, CMV, and polyhedrin promoters are also appropriate. The particular promoter is selected based on the host or host cell. In addition, promoters that are inducible by methanol, copper sulfate, galactose, by low phosphate,

by alcohol, *e.g.*, ethanol, for example, may also be used and are well known in the art.

A nucleic acid that encodes OXO may optionally comprise heterologous sequences. For example, a secretion sequence is included at the N-terminus of an OXO polypeptide in some embodiments. Signal sequences, such as these from α Mating Factor, BGL2, yeast acid phosphatase (PHO), xylanase, alpha amylase, from other yeast secreted proteins, and secretion signal peptides derived from other species that are capable of directing secretion from the host cell, may be useful. Similarly other heterologous sequences such as linkers (*e.g.*, comprising a cleavage or restriction endonuclease site) and one or more expression control elements, an enhancer, a terminator, a leader sequence, and one or more translation signals are within the scope of this description. These sequences may optionally be included in a construct and/or linked to the nucleic acid that encodes OXO. Unless otherwise specified, "linked" sequences can be directly or indirectly associated with one another.

Similarly, an epitope or affinity tag such as Histidine, HA (hemagglutinin peptide), maltose binding protein, AviTag®, FLAG, or glutathione-S-transferase may be optionally linked to the OXO polypeptide. A tag may be optionally cleavable from the OXO after it is produced or purified. A skilled artisan can readily select appropriate heterologous sequences, for example, match host cell, construct, promoter, and/or secretion signal sequence.

OXO homologs or variants differ from an OXO reference sequence by one or more residues. Structurally similar amino acids can be substituted for some of the specified amino acids, for example. Structurally similar amino acids include: (I,L,V); (F,Y); (K,R); (Q,N); (D,E); and (G,A). Deletion, addition, or substitution of amino acids is also encompassed by the OXO homologs described herein. Such homologs and variants include polymorphic variants and natural or artificial mutants, as well as modified polypeptides in which one or more residues is modified, and mutants comprising one or more modified residues.

An OXO polypeptide or nucleic acid is "homologous" (or is a "homolog") if it is at least 40%, 50%, 60%, 70%, 75%, 80%, 85%, 90%, 95%, 97%, 98%, 99%, or 100% identical to a reference sequence. If the homolog is not identical to the reference sequence, it is a "variant." A homolog is "substantially identical" to a reference OXO sequence if the nucleotide or amino acid sequence of the homolog differs from the reference sequence (*i.e.*, by truncation, deletion, substitution, or addition) by not more than 1,2, 3, 4, 5, 8,10, 20, or 50 residues, and retains (or encodes a polypeptide that retains) the ability to catalyze the oxidation of oxalate. Fragments of an oxalate oxidase may be homologs, including variants and/or substantially identical sequences. By way of example, homologs may be derived from various sources of OXO, or they may be derived from or related to a reference sequence by truncation, deletion, substitution, or addition mutation. Percent identity between two nucleotide or amino acid sequences may be determined by standard alignment algorithms such as, for example, Basic Local Alignment Tool (BLAST) described in Altschul et al., J. Mol. Biol., 215: 403-410 (1990), the algorithm of Needleman et al., J. Mol. Biol., 48:444-453 (1970), or the algorithm of Meyers et al., Comput. Appl. Biosci. 4:11-17 (1988). Such algorithms are incorporated into the BLASTN, BLASTP, and "BLAST 2 Sequences" programs (see www.ncbi.nhn.nih.gov/BLAST). When utilizing such programs, the default parameters can be used. For example, for nucleotide sequences the following settings can be used for "BLAST 2 Sequences": program BLASTN, reward for match 2, penalty for mismatch -2, open gap and extension gap penalties 5 and 2 respectively, gap x_dropoff 50, expect 10, word size 11, filter ON. For amino acid sequences the following settings can be used for "BLAST 2 Sequences": program BLASTP, matrix BLOSUM62, open gap and extension gap penalties 11 and 1 respectively, gap x_dropoff 50, expect 10, word size 3, filter ON. The amino acid and nucleic acid sequences for OXOs that are appropriate to form the crystals described herein, may include homologous, variant, or substantially identical sequences.

## Purification of Oxalate Oxidase

Oxalate oxidase proteins or polypeptides may be purified from the source, such as a natural or recombinant source, prior to crystallization. A polypeptide that is referred to herein as "isolated" is a polypeptide that is substantially free of its natural environment, such as proteins, lipids, and/or nucleic acids of their source of origin *(e.g.,* cells, tissue (*i.e.*, plant tissue), or fluid or medium (in the case of a secreted polypeptide)). Isolated polypeptides include those obtained by methods described herein or other suitable methods, and include polypeptides that are substantially pure or essentially pure, and polypeptides produced by chemical synthesis, by recombinant production, or by combinations of biological and chemical methods. Optionally, an isolated protein has undergone further processing after its production, such as by purification steps.

Purification may comprise buffer exchange and chromatographic steps. Optionally, a concentration step may be used, *e.g.*, by dialysis, chromatofocusing chromatography, and/or associated with buffer exchange. In certain instances, cation exchange chromatography is used for purification, including sulfopropyl Sepharose chromatography or a CM52 or similar cation exchange column. Buffer exchange optionally precedes chromatographic separation, and may be performed by tangential flow filtration such as diafiltration. In certain preparations, OXO is at least 70%, 80%, 85%, 90%, 95%, 96%, 97%, 98%, 99%, 99.5%, 99.7%, or 99.9% pure.

Purification in gram-scale runs is appropriate to prepare OXO, and procedures are optimized for efficient, inexpensive, manufacturing-scale OXO purification. For example, purification of at least 0.5, 1, 2, 5, 10, 20, 50, 100,

500, or 1000 grams or more of OXO in a purification procedure is provided. In one exemplary procedure, tangential flow filtration of starting samples of at least 1OL, 50L, 100L, 500L, 1000L or more is provided, allowing buffer exchange and precipitation of contaminate proteins. A single SP-sepharose column is optionally used for purification of OXO.

Crystallization of purified OXO may also remove contaminants, for example to further purify OXO preparations. For example, OXO cystallized as described in Example 6, has reduced levels of low molecular weight contaminants, as compared to soluble purified OXO. In some aspects, contaminants having a measured mass (by matrix assisted laser desorption ionization mass spectroscopy (MALDI-MS)) of 0-10 KDa, 1-10 KDa, 0.5-5 KDa, or 2-5 KDa are selectively excluded from the crystal form. For example, MALDI-MS analysis of OXO purified by diafiltration and SP-Sepharose and crystallized by the large scale crystallization procedure described in part (a) of Example 6, demonstrates that contaminants with measured masses of approximately 2.5, 3.0, 3.7, 3.8, 4.0, 4.2, and 5.0 KDa are substantially removed by crystallization. Purification by crystallization may also be done using, e.g., crude oxalate oxidase containing fermentation media.

## Crystallization of Oxalate Oxidase

Oxalate oxidase crystals can be prepared using an OXO polypeptide, such as a hexamer, as described above. *See,* Woo et al., FEBS Letters 437:87-90 (1998); Woo et al., Nature Struct. Biol. 7:1036-1040 (2000). Vapor diffusion (such as, *e.g.*, hanging drop and sitting drop methods), and batch methods of crystallization, for example, can be used. Oxalate oxidase crystals may be grown by controlled crystallization of the protein out of an aqueous solution or an aqueous solution that includes organic solvents. Conditions to be controlled include the rate of evaporation of solvent, the presence of appropriate co-solutes and buffers, pH, and temperature, for example.

For therapeutic administration, such as to treat a condition or disorder related to oxalate levels, a variety of OXO crystal sizes are appropriate. In certain embodiments, crystals of less than about 500 μm average dimension are administered. Oxalate oxidase crystals with an average, maximal, or minimal dimension (for example) that is about 0.01, 0.1, 1, 5, 10, 25, 50, 100, 200, 300, 400, 500, or 1000 μm in length are also provided. Microcrystalline showers are also suitable.

Ranges are appropriate and would be apparent to the skilled artisan. For example, the protein crystals may have a longest dimension between about 0.01 μm and about 500 μm, alternatively, between 0.1 μm and about 50 μm. In a particular embodiment, the longest dimension ranges from about 0.1 μm to about 10 μm. Crystals may also have a shape chosen from spheres, needles, rods, plates, such as hexagons and squares, rhomboids, cubes, bipryamids and prisms. In illustrative embodiments, the crystals are cubes having a longest dimension of less than 5 μm. *See,* for example, Figures 5-7.

In general, crystals are produced by combining the protein to be crystallized with an appropriate aqueous solvent or aqueous solvent containing appropriate crystallization agents, such as salts or organic solvents. The solvent is combined with the protein and optionally subjected to agitation at a temperature determined experimentally to be appropriate for the induction of crystallization and acceptable for the maintenance of protein activity and stability. The solvent can optionally include co-solutes, such as divalent cations, co-factors or chaotropes, as well as buffer species to control pH. The need for co-solutes and their concentrations are determined experimentally to facilitate crystallization. In an industrial-scale process, the controlled precipitation leading to crystallization can be carried out by the combination of protein, precipitant, co-solutes and, optionally, buffers in a batch process, for example. Alternative laboratory crystallization methods and conditions, such as dialysis or vapor diffusion, can be adopted (McPherson, et al., Methods Enzymol. 114:112-20 (1985) and Gilliland, J. Crystal Growth 90:51-59 (1998)). Occasionally, incompatibility between the cross-linking agent and the crystallization medium might require changing the buffers (solvent) prior to cross-linking.

As set forth in the Examples, oxalate oxidase crystallizes under a number of conditions, including a wide pH range (*e.g.*, pH 3.5 to 8.0). A precipitant such as a low molecular polyethylene glycol (such as, *e.g.*, PEG 600, PEG 400, PEG 200) or an organic cosolvent such as 2-methyl-2,4-pentanediol (MPD) is included in some embodiments as described. Common salts that may also be used are sodium chloride and zinc acetate.

Oxalate oxidase may be at a concentration of, *e.g.*, at least 5, 10, 15, 20, 25, 3 0, 35, 40, 45, or 50, 60, 70, 80, 90, or 100 mg/ml, or more in a crystallization solution. The efficiency or yield of a crystallization reaction is at least 50%, 60%, 70%, 80%, 90%, 91%, 92%, 93%, 94%, 95%, 96%, 97%, 98%, 99%, or more. In one embodiment, crystals of oxalate oxidase are grown or produced by a batch process by mixing a solution of oxalate oxidase with an appropriate buffer. In certain embodiments, the buffer is 65 mM citrate-phosphate buffer, pH 3.5 and 28.5% PEG 600.

## Stabilized crystals

Once oxalate oxidase crystals have been grown in a suitable medium they are stabilized by cross-linking. Cross-linking results in stabilization of the crystal lattice by introducing covalent links between the constituent protein molecules of the crystal. This makes possible transfer of the protein into an alternate environment that might otherwise

be incompatible with the existence of the crystal lattice or even with the existence of intact protein. Oxalate oxidase crystals may be cross-linked through, *e.g.*, lysine amine groups, thiol (sulfhydryl) groups, and carbohydrate moieties. Cross-linked crystals are also referred to as CLEC OXO, or CLEC herein.

A cross-linked crystal may alter the enzymatic stability (*e.g.*, pH, temperature, mechanical and/or chemical stability), the pH profile of OXO activity, the solubility, the uniformity of crystal size or volume, the rate of release of enzyme from the crystal, and/or the pore size and shape between individual enzyme molecules in the underlying crystal lattice.

Advantageously, cross-linking or stabilizing according to the present invention is carried out in such a way that the crystals comprise an OXO that shows at least 30%, 40%, 50%, 60%, 70%, 80%, 90%, 95%, 96%, 97%, 98%, 99%, 99.5%, 99.7%, or 99.9% or more of the activity as compared to unmodified OXO. Stability may be increased by at least 30%, 40%, 50%, 60%, 70%, 80%, 90%, 100%, 150%, 200%, 250%, 300% or more as compared to unmodified OXO. Stability can be measured under conditions of storage, such as pH stability, temperature stability, stability against gut proteases, dissolution stability, and as in vivo biological stability, for example.

In certain instances, cross-linking slows the dissolution of the OXO polypeptides in the crystal into solution, effectively immobilizing the protein molecules into microcrystalline particles. Upon exposure to a trigger in the environment surrounding the cross-linked protein crystals, such as under conditions of use rather than storage, the protein molecules slowly dissolve, releasing active OXO polypeptide and/or increasing OXO activity. The rate of dissolution is controlled, for example, by one or more of the following factors: the degree of cross-linking, the length of time of exposure of protein crystals to the cross-linking agent, the rate of addition of cross-linking agent to the protein crystals, the nature of the cross-linker, the chain length of the cross-linker, pH, temperature, presence of sulfahydryl reagents like cysteine, gluthathione, the surface area of the cross-linked protein crystals, the size of the cross-linked protein crystals, and the shape of the cross-linked protein crystals.

Cross-linking can be achieved using one or a combination of a wide variety of cross-linking agents, including a multifunctional agent, at the same time (in parallel) or in sequence. Upon exposure to a trigger in the surrounding environment, or over a given period of time, the cross-links between protein crystals cross-linked with such multifunctional cross-linking agents lessen or weaken, leading to protein dissolution or release of activity. Alternatively, the cross-links may break at the point of attachment, leading to protein dissolution or release of activity. *See* U.S. Patent Nos. 5,976,529 and 6,140,475.

In some embodiments the cross-linking agent is a multifunctional cross-linking agent having at least 2, 3, 4, 5, or more active moieties. In various embodiments, the agent may be chosen from glutaraldehyde, succinaldehyde, octanedialdehyde, glyoxal, dithiobis(succinimidylpropionate), 3,3'-dithiobis(sulfosuccinimidylpropionate), dimethyl 3,3'-dithiobispropionimidate·HCl, *N*-succinimidyl-3-(2-pyridyldithio)propionate, hexamethylenediamine, diaminooctane, ethylenediamine, succinic anhydride, phenylglutaric anhydride, salicylaldehyde, acetimidate, formalin, acrolein, succinic semialdehyde, butyraldehyde, dodecylaldehyde, glyceraldehyde, and *trans*-oct-2-enal.

Additional multifunctional cross-linking agents include halo-triazines, *e.g.*, cyanuric chloride; halo-pyrimidines, *e.g.*, 2,4,6-trichloro/bromo-pyrimidine; anhydrides or halides of aliphatic or aromatic mono- or di-carboxylic acids, *e.g.*, maleic anhydride, (meth)acryloyl chloride, chloroacetyl chloride; N-methylol compounds, *e.g.*, N-methylol-chloro acetamide; di-isocyanates or di-isothiocyanates, *e.g.*, phenylene-1,4-di-isocyanate and aziridines. Other cross-linking agents include epoxides, such as, for example, di-epoxides, tri-epoxides and tetra-epoxides. In one embodiment of this invention, the cross-linking agent is glutaraldehyde, a bifunctional agent, and glutaraldehyde is used alone or in sequence with an epoxide. Other cross-linking reagents *(see,* for example, the 1996 catalog of the Pierce Chemical Company) may also be used, at the same time (in parallel) or in sequence with reversible cross-linking agents, such as those described below.

According to an alternate embodiment of this invention, cross-linking may be carried out using reversible cross-linking agents, in parallel or in sequence. The resulting cross-linked protein crystals are characterized by a reactive multi-functional linker, into which a trigger is incorporated as a separate group. The reactive functionality is involved in linking together reactive amino acid side chains in a protein and the trigger consists of a bond that can be broken by altering one or more conditions in the surrounding environment (*e.g.*, pH, presence of reducing agent, temperature, or thermodynamic water activity).

The cross-linking agent may be homofunctional or heterofunctional. The reactive functionality (or moiety) may, *e.g.*, be chosen from one of the following functional groups (where R, R', R", and R"' may be alkyl, aryl or hydrogen groups):

I. Reactive acyl donors, such as, *e.g.*: carboxylate esters RCOOR', amides RCONHR', Acyl azides $RCON_3$, carbodiimides R-N=C=N-R', N-hydroxyimide esters, RCO-O-NR', imidoesters R-C=$NH2^+$ (OR'), anhydrides RCO-O-COR', carbonates RO-CO-O-R', urethanes RNHCONHR', acid halides RCOHal (where Hal=a halogen), acyl hydrazides RCONNR'R", and O-acylisoureas RCO-O-C NR'(-NR"R"')

II. Reactive carbonyl groups, such as, *e.g.*: aldehydes RCHO and ketones RCOR', acetals $RCO(H_2)R'$, and ketals $RR'CO_2$ R'R" (Reactive carbonyl containing functional groups known to those well skilled in the art of protein immobilization and cross-linking are described in the literature (Pierce Catalog and Handbook, Pierce Chemical Company, Rockford, Ill. (1994); S. S. Wong, Chemistry of Protein Conjugation and Cross-Linking, CRC Press, Boca Raton, Fla. (1991));

III. Alkyl or aryl donors, such as, *e.g.*: alkyl or aryl halides R-Hal, azides R-$N_3$, sulfate esters $RSO_3$ R', phosphate esters RPO(OR'$_3$), alkyloxonium salts $R_3$ O+, sulfonium $R_3$ S+, nitrate esters $RONO_2$, Michael acceptors RCR'=CR"'COR", aryl fluorides ArF, isonitriles R$\overset{+}{N}$ ≡ C - , haloamines $R_2$ N-Hal, alkenes, and alkynes;

IV. Sulfur containing groups, such as, *e.g.*: disulfides RSSR', sulfhydryls RSH, and epoxides

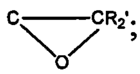

and

V. Salts, such as, e.g.: alkyl, or aryl ammonium salts $R_4$ N+, carboxylate RCOO-, sulfate $ROSO_3$-, phosphate $ROPO_3$-, and amines $R_3$ N-.

Reversible cross-linking agents, for example, comprise a trigger. A trigger includes an alkyl, aryl, or other chain with activating group that can react with the protein to be cross-linked. Those reactive groups can be any variety of groups such as those susceptible to nucleophilic, free radical or electrophilic displacement including halides, aldehydes, carbonates, urethanes, xanthanes, epoxides among others. For example, reactive groups may be labile to acid, base, fluoride, enzyme, reduction, oxidation, thiol, metal, photolysis, radical, or heat.

Additional examples of reversible cross-linking agents are described in T. W. Green, Protective Groups in Organic Synthesis, John Wiley & Sons (Eds.) (1981). Any variety of strategies used for reversible protecting groups can be incorporated into a cross-linker suitable for producing cross-linked protein crystals capable of reversible, controlled solubilization. Various approaches are listed, in Waldmann's review of this subject, in Angewante Chemise Inl. Ed. Engl., 35:2056 (1996).

Other types of reversible cross-linking agents are disulfide bond-containing cross-linkers. The trigger breaking cross-links formed by such cross-linking agents is the addition of reducing agent, such as cysteine, to the environment of the cross-linked protein crystals. Exemplary disulfide cross-linking agents are described in the Pierce Catalog and Handbook (1994-1995). Examples of such cross-linkers and methods are disclosed in U.S. Patent No. 6,541,606, relevant portions of which are incorporated by reference.

In addition, cross-linking agents which cross-link between carbohydrate moieties or between a carbohydrate moiety and an amino acid may also be used.

To form cross-linked crystals, the concentration of the cross-linking agent may be, *e.g.*, about 0.5%, 1%, 2%, 3%, 3.5%, 4%, 5%, 6%, 7%, 8%, 9%, 10%, 15%, or 20% wt/v in solution. It may be necessary to exchange buffers prior to cross-linking. Crystals, including CLECs, may be optionally lyophilized or otherwise formulated.

The crystals, including the cross-linked crystals, and compositions comprising those cross-linked crystals described herein are useful in the methods of treatment and methods to reduce oxalate levels described herein.

## Compositions

OXO cross-linked crystals, are provided as a composition, such as a pharmaceutical composition (*see, e.g.,* U.S. Patent No. 6,541,606, describing formulations and compositions of protein crystals). Pharmaceutical compositions comprising OXO crystals comprise the OXO crystal with one or more ingredients or excipients, including, but not limited to sugars and biocompatible polymers. Examples of excipients are described in Handbook of Pharmaceutical Excipients, published jointly by the American Pharmaceutical Association and the Pharmaceutical Society of Great Britain, and further examples are set forth below.

The OXO enzyme may be administered as a crystal in a composition as any of a variety of physiologically acceptable salt forms, and/or with an acceptable pharmaceutical carrier and/or additive as part of a pharmaceutical composition. Physiologically acceptable salt forms and standard pharmaceutical formulation techniques and excipients are well known to persons skilled in the art (*see, e.g.,* Physician's Desk Reference (PDR) 2003, 57th ed., Medical Economics Company, 2002; and Remington: The Science and Practice of Pharmacy, eds. Gennado et al., 20th ed, Lippincott, Williams & Wilkins, 2000). For the purposes of this application, "formulations" include "crystal formulations."

Oxalate oxidase useful in the methods of this invention may be combined with an excipient. According to this invention, an "excipient" acts as a filler or a combination of fillers used in pharmaceutical compositions. Exemplary ingredients and excipients for use in the compositions are set forth as follows.

*Biocompatible polymers, i.e.,* polymers that are non-antigenic (when not used as an adjuvant), non-carcinogenic, non-toxic and which are not otherwise inherently incompatible with living organisms may be used in the OXO crystal compositions described herein. Examples include: poly (acrylic acid), poly (cyanoacrylates), poly (amino acids), poly (anhydrides), poly (depsipeptide), poly (esters) such as poly (lactic acid) or PLA, poly (lactic-co-glycolic acid) or PLGA, poly (β-hydroxybutryate), poly (caprolactone) and poly (dioxanone); poly (ethylene glycol), poly ((hydroxypropyl) methacrylamide, poly [(organo)phosphazene], poly (ortho esters), poly (vinyl alcohol), poly (vinylpyrrolidone), maleic anhydride-alkyl vinyl ether copolymers, pluronic polyols, albumin, alginate, cellulose and cellulose derivatives, collagen, fibrin, gelatin, hyaluronic acid, oligosaccharides, glycaminoglycans, sulfated polysaccharides, blends and copolymers thereof.

*Biodegradable polymers, i.e.*, polymers that degrade by hydrolysis or solubilization may be included in OXO crystal compositions. Degradation can be heterogenous -- occurring primarily at the particle surface, or homogenousdegrading evenly throughout the polymer matrix.

*Ingredients* such as one or more excipients or pharmaceutical ingredients or excipients may be included in OXO crystal compositions. An ingredient may be an inert or active ingredient.

**Methods of Treating Oxalate-Related Disorders with OXO Crystals**

The cross-linked crystals of oxalate oxidase are for use in a mammalian subject to treat, prevent, or reduce the risk of occurrence of a condition associated with elevated levels of oxalate. The elevated levels of oxalate may be detected, *e.g.*, in a biological sample from the subject, such as a body fluid, including urine, blood, serum, or plasma. In certain embodiments, urinary oxalate levels are detected.

In some embodiments, uses for treating hyperoxaluria in individuals with primary hyperoxaluria, enteric hyperoxaluria, hyperoxaluria caused by surgical intervention, idiopathic hyperoxaluria, oxalosis are provided. In other instances, elevated oxalate-related disorders of the kidneys, bone, liver gastrointestinal tract and pancreas are amenable to treatment with the uses disclosed herein. Further disorders or diseases treated by the uses provided herein include, but are not limited to ethylene glycol (oxalate) poisoning, idiopathic urinary stone disease, renal failure (including progressive, chronic, or end-stage renal failure), steatorrhoea, malabsorption, ileal disease, vulvodynia, cardiac conductance disorders, inflammatory bowel disease, cystic fibrosis, exocrine pancreatic insufficiency, Crohn's disease, ulcerative colitis, nephrocalcinosis, osteoporosis, urolithiasis, and nephrolithiasis. Such conditions and disorders may optionally be acute or chronic.

The medical uses claimed may reduce oxalate levels in a subject by at least 10%, 20%, 30%, 40%, 50%, 60%, 70%, 80%, 90%, 95%, or more as compared to levels in an untreated or control subject. In some embodiments, reduction is measured by comparing the oxalate level in a subject before and after administration of OXO. In some embodiments, the invention provides a use in a method of treating or ameliorating an oxalate-related condition or disorder, to allow one or more symptoms of the condition or disorder to improve by at least 10%, 20%, 30%, 40%, 50%, 60%, 70%, 80%, 90%, 95% or more. In certain embodiments the methods reduce levels of endogenous oxalate and/or adsorption of dietary oxalate.

In some embodiments, uses in methods for treating individuals having a genotype associated with high oxalate levels are provided, such as individuals homozygous or heterozygous for a mutation that reduces activity of, *e.g.*, alanine: glyoxalate aminotransferase, glyoxylate reductase/hydroxypyruvate reductase, hepatic glycolate oxidase, or another enzyme involved in oxalate metabolism or associated with hyperoxaluria. In other embodiments, uses in methods for treating individuals having low or no *Oxalobacter formigenes* enteric colonization are provided.

The disclosed methods include administering therapeutically effective amounts of oxalate oxidase to a mammalian subject at risk for, susceptible to, or afflicted with a condition associated with elevated levels of oxalate. The populations treated by the methods of the invention include, but are not limited to, subjects suffering from, or at risk for developing an oxalate-related disorder such as, *e.g.*, primary hyperoxaluria or enteric hyperoxaluria.

Subjects treated according to the methods of the invention include but are not limited to mammals, including humans, non-human primates, primates, baboons, chimpanzees, monkeys, rodents (*e.g.*, mice, rats), rabbits, cats, dogs, horses, cows, sheep, goats, pigs, etc.

**Indications, Symptoms, and Disease Indicators**

Many methods are available to assess development or progression of an oxalate-related disorder or a condition associated with elevated oxalate levels. Such disorders include, but are not limited to, any condition, disease, or disorder as defined above. Development or progression of an oxalate-related disorder may be assessed by measurement of

urinary oxalate, plasma oxalate, measurement of kidney or liver function, or detection of calcium oxalate deposits, for example.

A condition, disease, or disorder may be identified by detecting or measuring oxalate concentrations, for example in a urine sample or other biological sample or fluid. An early symptom of hyperoxaluria is typically kidney stones, which may be associated with severe or sudden abdominal or flank pain, blood in the urine, frequent urges to urinate, pain when urinating, or fever and chills. Kidney stones may be symptomatic or asymptomatic, and may be visualized, for example by imaging the abdomen by x-ray, ultrasound, or computerized tomography (CT) scan. If hyperoxaluria is not controlled, the kidneys are damaged and kidney function is impaired. Kidneys may even fail. Kidney failure (and poor kidney function) may be identified by a decrease in or no urine output (glomerular filtration rate), general ill feeling, tiredness, and marked fatigue, nausea, vomiting, anemia, and/or failure to develop and grow normally in young children. Calcium oxalate deposits in other tissues and organs may also be detected by methods including direct visualization (*e.g.* in the eyes), x-ray, ultrasound, CT, echocardiogram, or biopsy (*e.g.* bone, liver, or kidney).

Kidney and liver function, as well as oxalate concentrations, may also be assessed using well known direct and indirect assays. The chemical content or urine, blood or other biological sample may also be tested by well known techniques. For example, oxalate, glycolate, and glycerate levels may be measured. Assays for liver and kidney function are well known, such as, for example, the analysis of liver tissue for enzyme deficiencies and the analysis of kidney tissue for oxalate deposits. Samples may also be tested for DNA changes known to cause primary hyperoxaluria.

Other indications for treatment and include, but are not limited to, the presence of one or more risk factors, including those discussed previously and in the following sections. A subject at risk for developing or susceptible to a condition, disease, or disorder or a subject who may be particularly receptive to treatment with oxalate oxidase may be identified by ascertaining the presence or absence of one or more such risk factors, diagnostic, or prognostic indicators. Similarly, an individual at risk for developing an oxalate-related disorder may be identified by analysis of one or more genetic or phenotypic markers.

The methods disclosed are useful in subjects with urinary oxalate levels of at least 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, or 400 mg of oxalate per 24 hour period, or more. In certain embodiments, the oxalate level is associated with one or more symptoms or pathologies. Oxalate levels may be measured in a biological sample, such as a body fluid including blood, serum, plasma, or urine. Optionally, oxalate is normalized to a standard protein or substance, such as creatinine in urine. In some embodiments, the claimed methods include administration of oxalate oxidase to reduce circulating oxalate levels in a subject to undetectable levels, or to less than 1%, 2%, 5%, 10%, 20%, 30%, 40%, 50%, 60%, 70%, or 80% of the subject's oxalate levels prior to treatment, within 1, 3, 5, 7, 9, 12, or 15 days.

Hyperoxaluria in humans can be characterized by urinary oxalate excretion of greater than 40 mg (approximately 440 $\mu$mol) or 30 mg per day. Exemplary clinical cutoff levels are 43 mg/day (approximately 475 $\mu$mol) for men and 32 mg/day (approximately 350 $\mu$mol) for women, for example. Hyperoxaluria can also be defined as urinary oxalate excretion greater than 30 mg per day per gram of urinary creatinine. Persons with mild hyperoxaluria may excrete at least 30-60 or 40-60 mg of oxalate per day. Persons with enteric hyperoxaluria may excrete at least 80 mg of urinary oxalate per day, and persons with primary hyperoxaluria may excrete at least 200 mg per day, for example. *See* (Shekarriz, www.emedicine.com/med/topic3027.htm).

## Administration of The Compounds And Compositions

Administration of oxalate oxidase in accordance with the methods of the invention is not limited to any particular delivery system and includes, administration via the upper gastointestinal tract, *e.g.*, the mouth (for example in capsules, suspension, tablets, or with food), or the stomach, or upper intestine (for example by tube or injection) to reduce oxalate levels in an individual. In certain cases, the OXO is administered to reduce endogenous oxalate levels and/or concentrations.

Administration to an individual may occur in a single dose or in repeat administrations, and in any of a variety of physiologically acceptable forms, and/or with an acceptable pharmaceutical carrier and/or additive as part of a pharmaceutical composition (described earlier). In the disclosed methods, oxalate oxidase may be administered alone, concurrently or consecutively over overlapping or nonoverlapping intervals with one or more additional biologically active agents, such as, *e.g.*, pyridoxine (vitamin B-6), orthophosphate, magnesium, glycosaminoglycans, calcium, iron, aluminum, magnesium, potassium citrate, cholestyramine, organic marine hydrocolloid, plant juice, such as, e.g., banana stem juice or beet juice, or L-cysteine. Biologically active agents that reduce oxalate levels or that increase the activity or availability of OXO are provided. In sequential administration, the oxalate oxidase and the additional agent or agents may be administered in any order. In some embodiments, the length of an overlapping interval may be more than 2, 4, 6, 12, 24, 48 weeks or more.

The oxalate oxidase may be administered as the sole active compound or in combination with another active compound or composition. Unless otherwise indicated, the oxalate oxidase is administered as a dose of approximately

from 10 μg/kg to 25 mg/kg or 100 mg/kg, depending on the severity of the symptoms and the progression of the disease. The appropriate therapeutically effective dose of OXO is selected by a treating clinician and would range approximately from 10 μg/kg to 20 mg/kg, from 10 μg/kg to 10 mg/kg, from 10 μg/kg to 1 mg/kg, from 10 μg/kg to 100 μg/kg, from 100 μg/kg to 1 mg/kg, from 100 μg/kg to 10 mg/kg, from 500 μg/kg to 5 mg/kg, from 500 μg/kg to 20 mg/kg, from 1 mg/kg to 5 mg/kg, from 1 mg/kg to 25 mg/kg, from 5 mg/kg to 100 mg/kg, from 5 mg/kg to 50 mg/kg, from 5 mg/kg to 25 mg/kg, and from 10 mg/kg to 25 mg/kg. Additionally, specific dosages indicated in the Examples or in the Physician's Desk Reference (PDR) 2003, 57th ed., Medical Economics Company, 2002, may be used.

The following examples provide illustrative embodiments of the invention. One of ordinary skill in the art will recognize the numerous modifications and variations that may be performed without altering the scope of the present invention. Such modifications and variations are encompassed within the scope of the invention. The Examples do not in any way limit the invention.

## EXAMPLES

### Example 1: Recombinant Production of Oxalate Oxidase

*In Human Embryonic Kidney (HEK293) cells:* DNA encoding OXO is cloned into a suitable expression vector. After sequence confirmation, the vector can be linearized and transformation of the linearized vector to pre-seeded HEK293 cells may be carried out using Lipofectamine™ 2000 Transfection Reagent in a 6 cm diameter dish. After culturing approximately overnight after the transfection in appropriate medium, transformants are selected in medium supplemented with 0.5 g/L of neomycin. Stably transfected HEK293 cell clones are identified after growth in neomycin containing medium for up to 3 weeks. The clones are then isolated and propagated, and used for OXO expression.

*In Chinese Hamster Ovary (CHO) cells:* DNA encoding OXO gene is cloned into a suitable expression vector. Cultured CHO lec 3.2.8.1 cells are then detached by trypsin digestion and harvested by centrifugation. The cells are then suspended in Electroporation phosphate buffered saline buffer (EPBS) to a final concentration of ~1 x $10^7$/ml, and transformed with the linearized vector by electroporation. After overnight culture, exchange the medium to medium supplemented with 0.5 g/L of neomycin and keep exchanging the medium to screen for the stable transfected CHO cell clones. Once the stable transfected cell clones are established and propagated, these cells are used for OXO expression.

*In Pichia Pastoris:* DNA encoding OXO gene, for example SEQ ID NO:1, is cloned into a suitable expression vector. After sequence confirmation, the vector can be linearized then transformed into a *Pichia Pastoris* host cell *(see,* Whittaker et al., J. Biol. Inorg. Chem. 7:136-145 (2002)). Transformants are selected with Zeocin, expanded in buffered glycerol-complex medium (*BMGY*), and induced with methanol. OXO may then be isolated from the culture medium.

*In Saccharomyces cervisiae:* The synthetic OXO gene described (SEQ ID NO:1) above may also be cloned into a suitable expression vector containing, *e.g.*, the Gal1 promoter (pGa1) and the terminator for expression. After sequence confirmation, the expression vector is transformed into the competent *Saccharomyces cerevisiae* W303-1A by electroporation. The transformants are screened and propagated before use for OXO expression.

*In insect cells:* DNA encoding OXO may be cloned into a suitable expression vector, such as, *e.g.*, a baculovirus system. After sequence confirmation, the vector may be transformed into competent DH10Bac *E. coli* cells, and *E. coli* cells containing the recombinant bacmid screened and verified. The recombinant bacmid DNA is isolated and used to transfect insect Sf9 cells using reagents such as Cellfectin reagent. The recombinant baculovirus particles can then be isolated, propagated, and tittered before use to infect Sf9 cells for OXO expression.

*In E coli:* DNA encoding OXO is cloned into a suitable the *E coli* expression vector. After sequence confirmation, the vector is transformed into competent *E. coli* Origami B (DE3), which allows the formation of disulfide bonds in the recombinant protein expressed in this strain. The transformants are screened by growing the transformants on nutrient plates containing antibiotics and verified by colony PCR using OXO gene specific primers. The transformants are then cultured in the liquid medium and induced with isopropyl-beta-D-thiogalactopyranoside (IPTG) for OXO expression.

### Example 2: Purification of OXO

*OXO* was purified from 5 L of pooled expression medium [from where?] and diafiltered against 20 mM phosphate buffer (pH 7.0). After 10-fold concentration of the expression medium, 100 ml of a 50% slurry of DE52 anion exchanger resin was added, and the mixture was stirred for 1 hour at 4 °C. Oxalate oxidase does not bind to the DE52 resin which was separated by centrifugation. The medium, was than diafiltered against 50 mM succinate buffer (pH 4.5). The diafiltered preparation was then loaded onto a SP Sepharose™ cation exchange column, from which bound OXO was eluted with a linear gradient of 1 M ammonium sulfate in 50 mM succinate buffer. Each fraction was assayed for oxalate oxidase activity; active fractions were pooled. *See* Table 1.

Table 1: Recombinant OXO Purification from shake flask

| | Volume (ml) | Concentration (mg/ml) | Total Protein (mg) | Specific Activity (unit/mg) | Yield (%) |
|---|---|---|---|---|---|
| Expression Medium | 6000 | 5.68 | 34,075 | 0.07 | 100 |
| Post TFF Pool | 460 | 7.18 | 3,304 | 0.61 | 70.8 |
| DE52 Unbound | 390 | 4.32 | 1,685 | 0.58 | 67.5 |
| SP Fraction Pool | 24 | 2.59 | 62 | 6.88 | 41.8 |

Recombinant oxalate oxidase from barley purified from yeast expression medium exhibits a specific activity of about 7-12 U/mg under standard assay conditions (Example 11).

## Example 3: Purification of OXO (Large Scale)

*Method A:* Expression medium from a 400 L yeast culture was diafiltered against 20 mM phosphate buffer (pH 7.0) and concentrated to a volume of about 20 L. The concentrated medium was stored frozen until purification. The purification of oxalate oxidase was carried out by diafiltration of 9560 ml concentrated expression medium *against* 50 mM citrate phosphate buffer (pH 4.0) and subsequent concentration to a volume of 2520 ml. Protein contaminants that precipitated during the process were removed by centrifugation. The supernatant was filtered through a 0.22 μm filter prior to loading onto a 5x45 cm SP Sepharose™ cation exchange column. Bound OXO was eluted with linear gradient of 0-100% 50 mM citrate phosphate buffer (pH 8.0) over six bed volumes. Over 4.7 g of purified oxalate oxidase was obtained with this two-step procedure, with a yield of 53%. The purification process was summarized in Table 2. The purified OXO has a,specific activity of 10.7 U/mg.

Table 2: Recombinant OXO Purification from shake flask

| | Volume (ml) | Concentration (mg/ml) | Total Protein (mg) | Specific Activity (unit/mg) | Yield (%) |
|---|---|---|---|---|---|
| Expression Medium | 9560 | 1.07 | 10191 | 9.48 | 100 |
| Post TFF Pool | 2520 | 3.09 | 7797 | 10.67 | 86.2 |
| SP Sepharose Column Pool | 2450 | 2.16 | 5280 | 9.48 | 51.8 |
| Conc. Pool | 226 | 21.11 | 4771 | 10.73 | 53.0 |

*Method B: Cell-free fermentation* broth containing secreted recombinant oxalate oxidase was concentrated to approximately 4 L from 16 L using a Tangential Flow Filtration (TFF)/ Centramate cassette and a Pall manifold, and subsequently diluted 5-fold with 50 mM citrate phosphate buffer (pH 4). This procedure was repeated four times. The medium was concentrated to a final volume of approximately 6.5 L, and then centrifuged at 9500 rpm to separate precipitated proteins. The supernatant was filtered through a 0.45 μm Sartobran capsule using a peristaltic pump to remove unwanted debris.

The filtered supernatant was loaded onto a SP Sepharose™ column (1 L packed bed volume) equilibrated with 50 mM citrate phosphate buffer (pH 4) using the AKTA FPLC system. Unbound proteins were washed with 3 column volumes (CV) of 50 mM citrate phosphate (pH 4.0). A linear pH gradient (pH 4.0-8.0) with a flow rate of 20 mL/minute was performed over a course of 6 CV; 15 mL fractions were collected. Oxalate oxidase eluted as a single peak with 50 mM citrate phosphate buffer at ~pH 6.0. The column was then washed with 2 CV 50 mM citrate phosphate buffer (pH 8.0).

Eluted *fractions* were analyzed by assaying for oxalate oxidase activity as described in Example 15. Active fractions were pooled in a 2 L bottle and concentrated to approximately 400 mL (approximately 20 mg/mL) with a Pellicon XL Biomax 10 TFF system.

Purity of the pooled fractions was assessed by SDS-PAGE and enzymatic activity by an oxalate oxidase activity assay. Samples were denatured by adding 5 μL sample buffer containing 5.3% 2-mercaptoethanol and heated to 99 °C for 5 min. The electrophoresis was conducted at a constant 120 V for approximately 2 hours. Gels were stained with Coomassie blue dye, and scanned using a Microtek ScanMaker 4 scanner/ Adobe software. Protein concentration was

determined using the Bradford method. Table 3 shows that the purification process was very efficient, with a recovery greater than 79%.

Table 3: Recombinant OXO Purification from shake flask

| | Volume (ml) | Concentration (mg/ml) | Total Protein (mg) | Specific Activity (unit/mg) | Yield (%) |
|---|---|---|---|---|---|
| Expression Medium | 16000 | 0.794 | 12,704 | 8.41 | 100.0 |
| Post TFF Pool, pre-0.45 μm filtration | 6560 | 1.982 | 13,002 | 8.75 | 106.5 |
| SP Sepharose™ Column Load | 6950 | 2.080 | 14,456 | 7.23 | 97.8 |
| SP Sepharose™ Column Pool | 2580 | 3.075 | 7,934 | 10.98 | 81.6 |
| Final | 410 | 17.290 | 7,089 | 11.96 | 79.4 |

**Example 4: Crystallization of OXO (Vapor Diffusion)**

*Hanging Drop Crystallization:* Hanging drop crystallization trials were performed using commercially available sparse matrix crystallization kits: Crystal Screen (Hampton Research; Aliso Viejo, CA), Crystal Screen 2 (Hampton Research), Wizard I (Emerald Biosystems; Bainbridge Island, WA), Wizard II (Emerald Biosystems), Cryo I (Emerald Biosystems), and Cryo II (Emerald Biosystems).

600 μl of reagent was placed each well. 3 μl of reagent was dispensed onto a glass microscope coverslip and 3 μl of oxalate oxidase dispensed into the reagent drop with minimal mixing. Up to five more drops were made from this 6 μl reagent and oxalate oxidase drop. As the drops were minimally mixed, each of the subsequent (smaller) drops had a different and unknown ratio of protein to reagent, thereby increasing the likelihood of obtaining crystals in a short period of time. The hanging drops were examined for crystals under a microscope after overnight incubation at room temperature. A large number of crystallization conditions were obtained, as shown in Table 4.

Table 4: Crystallization conditions for oxalate oxidase in hanging drops

| OXO Concentration (mg/ml) | Precipitant | Description of Crystals |
|---|---|---|
| 27[1] | 30% (v/v) PEG 600, 0.1 M MES, pH 6.00, 5% (w/v) PEG 1000, 10% (v/v) glycerol | Rod crystals |
| 27[1] | 40% (v/v) PEG 600, 0.1 M phosphate-citrate, pH 4.20 | Cube |
| 27[1] | 40% (v/v) PEG 400, 0.1 M MES, pH 6.00, 5% (w/v) PEG 3000 | Cube and Rods |
| 27[1] | 20% (w/v) PEG 8000, 0.1 M phosphate-citrate, pH 4.20, 0.2 M NaCl | Big rectangles |
| 10[1] | 10% (w/v) PEG 8000, 0.1 M MES, pH 6.00, 0.1 M Zn $(OAC)_2$ | Triangles |
| 10[1] | protein:reagent ratio =1:1 40% (v/v) PEG 600, 0.1 M CHES, pH 9.50 | Diamonds |
| 10[1] | protein:reagent ratio =1:1 10% (w/v) PEG 8000, 0.1 M MES, pH 6.00, 0.1 M $Zn(OAc)_2$ | Small rods |
| 10[1] | 40% (v/v) PEG 400, 0.1 M citrate, pH 5.50, 0.2 M $MgCl_2$ | Cubes |
| 10[1] | 40% (v/v) PEG 400, 0.1 M Na/phosphate, pH 6.20, 0.2 M NaCl | Very small cubes |
| 27[1] | 40% (v/v) PEG 400, 0.1 M Tris, pH 8.50, 0.2 M $LiS0_4$ | Rods |

(continued)

| OXO Concentration (mg/ml) | Precipitant | Description of Crystals |
|---|---|---|
| 27[1] | 40% PEG (v/v) PEG 600, 0.1 M imidazole, pH 8.00, 0.2 M $Zn(OAc)_2$ | Cubes |
| 27[1] | 50% (v/v) PEG 200, 0.1 M CHES, pH 9.50 | Huge oval crystals |
| 27[1] | 40% (v/v) PEG 400, 0.1 M HEPES, pH 7.50, 0.2 M $Ca(OAc)_2$ | Urchin crystals |
| 27[1] | 40% (v/v) PEG 300, 0.1 M phosphate-citrate, pH 4.20 | Rods and cubes |
| 27[1] | 40% PEG 600, 0.1 M CHES, pH9.50 | Small cubes |
| 27[1] | 50% (v/v) PEG 200, 0.1 M phosphate-citrate, pH 4.20, 0.2 M NaCl | Needles |
| 9.3[2] | 50% (v/v) PEG-400, CHES pH 9.5, 0.2 M NaCl | stars |
| 9.3[2] | 30% (v/v) PEG 600, 0.1 M MES, pH 6.00, 5% (w/v) PEG 1000, 10% (v/v) glycerol | diamond |
| 9.3[2] | 40% (v/v) PEG 400, 0.1 M Na/K phosphate, pH 6.20, 0.2 M NaCl | cubes |
| 9.3[2] | 40% (v/v) PEG 300, 0.1 M CHES, pH 9.50, 0.2 M NaCl | Long rods |
| 9.3[2] | 30% (v/v) PEG 600, 0.1 M HEPES, pH 7.50, 0.05 M $Li_2SO_4$, 10% glycerol | |
| 9.3[2] | 50% (v/v) PEG 200, 0.1 M Tris, pH 7.00, 0.05M $Li_2SO_4$ | cubes |
| 9.3[2] | 40% (v/v) PEG 400, 0.1 M Tris, pH 8.50, 0.2M Li2SO4 | Long rods |
| 9.3[2] | 40% (v/v) PEG 600, 0.1 M phosphate, pH 4.2 | cubes |
| 9.3[2] | 40% (v/v) PEG 300, 0.1 M HEPES, pH 7.50, 0.2 M NaCl | cubes |
| 9.3[2] | 40% (v/v) PEG 400, 0.1 M MES, pH 6.00, 5% (w/v) PEG 3000 | rhomboid |
| 9.3[2] | 40% (v/v) PEG 600, 0.1 M imidazole, pH 8.00, 0.2 M $Zn(OAc)_2$ | cube |
| 9.3[2] | 40% (v/v) PEG 400, 0.1 M citrate, pH 5.50, 0.2 M $MgCl_2$ | cube |
| 9.3[2] | 40% (v/v) PEG 300, 0.1 M phosphate-citrate, pH 4.20 | cube |
| 9.3[2] | 50% (v/v) PEG 200, 0.1 M CHES, pH 9.50 | 6-sided hexagon |
| 9.3[2] | 50% (v/v) PEG 200, 0.1 M Tris, pH 7.00 | Cube |
| 9.3[2] | 40% (v/v) PEG 300, 0.1 M imidazole, pH 8.00, 0.2 M $Zn(OAc)_2$ | cube |
| 9.3 | 30% (v/v) PEG 400, 0.1 M HEPES, pH 7.50, 5% (w/v) PEG 3000, 10% (v/v) glycerol | rhomboid |
| 9.3[2] | 40% (v/v) PEG 600, 0.1 M citrate, pH 5.50 | rods |
| 9.3[2] | 40% (v/v) PEG 600, 0.1 M CHES, pH 9.50 | rhomboids |
| 9.3[2] | 40% (v/v) PEG 400, 0.1 M acetate, pH 4.50 | cubes |
| 9.3[2] | 30% (v/v) PEG 600, 0.1 M Tris, pH 7.00, 0.5 M $(NH_4)_2SO_4$, 10% glycerol | stars |
| 9.3[2] | 40% (v/v) PEG 400, 0.1 M imidazole, pH 8.00 | Large rhomboid |
| 9.3[2] | 40% (v/v) PEG 300, 0.1 M acetate, pH 4.50, 0.2 M NaCl | cubes |

(continued)

| OXO Concentration (mg/ml) | Precipitant | Description of Crystals |
|---|---|---|
| 9.3[2] | 50% (v/v) PEG 200, 0.1 M phosphate-citrate, pH 4.20, 0.2 M NaCl | cubes |
| 9.3[2] | 40% (v/v) PEG 300, 0.1 M Tris, pH 7.00, 5% (w/v) PEG 1000 | cubes |
| 11.7[2] | 50% (v/v) PEG 400, 0.1 M CHES, pH9.50, 0.2 M NaCl | rods |
| 11.7[2] | 30% PEG 600, 0.1 M MES, pH 6.00,5% (w/v) PEG 1000, 10% (v/v) glycerol | Circular unsmooth surface |
| 11.7[2] | 40% (v/v) MPD, 0.1 M Tris, pH 7.0, 0.2 M $(NH_4)_2SO_4$ | cubes |
| 11.7[2] | 50% (v/v) PEG 200, 0.1 M acetate, pH 4.5 | Urchins haystack |
| 11.7[2] | 50% (v/v) PEG 200, 0.1 M Tris, pH 7.0, 0.05M $Li_2SO_4$ | Urchin haystack |
| 11.7[2] | 40% (v/v) PEG 300, 0.1 M cacodylate, pH 6.50, 0.2 M $Ca(OAc)_2$ | Plate rods |
| 11.7[2] | 40% (v/v) PEG 400, 0.1 M Tris, pH 8.50, 0.2 M $Li_2SO_4$ | Rods |
| 11.7[2] | 40% (v/v) PEG 600, 0.1 M phosphate-citrate, pH 4.2 | cube |
| 11.7[2] | 50% (v/v) PEG 200, 0.1 M cacodylate, pH 6.5, 0.2 M $Zn(OAc)_2$ | cube |
| 11.7[2] | 40% (v/v) PEG 400, 0.1 M MES, pH 6.00, 5% (w/v) PEG 3000 | cube |
| 11.7[2] | 50% (v/v) PEG 400, 0.1 M acetate, pH 4.5, 0.2 M $Li_2SO_4$ | oval |
| 11.7[2] | 40% (v/v) PEG 300, 0.1 M citrate, pH 4.5 | oval |
| 11.7[2] | 40% (v/v) PEG 300, 0.1 M phosphate-citrate, pH 4.2 | Oval + rods |
| 11.7[2] | 50% (v/v) PEG 200, 0.1 M CHES, pH 9.5 | oval |
| 11.7[2] | 40% (v/v) ethylene glycol, 0.1 M MES, pH 6.00, 0.2 M $Zn(OAc)_2$ | cubes |
| 11.7[2] | 40% (v/v) PEG 300, 0.1 M imidazole, pH 8.00, 0.2 M $Zn(OAc)_2$ | rods |
| 11.7[2] | 30% (v/v) PEG 400, 0.1 M HEPES, pH 7.5, 5% (w/v) PEG 3000, 10% (v/v) glycerol | rhomboids |
| 11.7[2] | 40% (v/v) PEG 400, 0.1 M acetate, pH 4.5 | cubes |
| 11.7[2] | 30% (v/v) PEG 600, 0.1 M Tris, pH 7.0, 0.5 M $(NH_4)_2SO_4$, 10% glycerol | stars |
| 11.7[2] | 40% PEG 400, 0.1 M HEPES, pH 7.5, 0.2 M $Ca(OAc)_2$ | urchin |
| 11.7[2] | 40% (v/v) PEG 300, 0.1 M acetate, pH 4.5, 0.2 M NaCl | cubes |
| 11.7[2] | 50% (v/v) PEG 200, 0.1 M phosphate-citrate, pH 4.2, 0.2 M NaCl | haystack |
| 11.7[2] | 40% PEG 300, 0.1 M Tris, pH 7.00, 0.2 M $(NH_4)_2SO_4$ | cube |
| 11.7[2] | 35% (v/v) 2-propanol, 0.1 M imidazole, pH 8,0.05M Zn $(OAc)_2$ | oval |
| 11.7[2] | 30% (v/v) PEG 400, 0.1 M CHES, pH 9.5 | sphere |
| 11.7[2] | 20% (w/v) PEG 1000, 0.1 M Tris, pH 8.5 | sphere |
| 11.7[2] | 20% (w/v) PEG 3000, 0.1 M imidazole, pH 8.0, 0.2 M $Zn(OAc)_2$ | plates |

(continued)

| OXO Concentration (mg/ml) | Precipitant | Description of Crystals |
|---|---|---|
| 11.7[2] | 2.0 M $(NH_4)2SO_4)$, 0.1 M phosphate-citrate, pH 4.2 | cubes |
| 11.7[2] | 20% (w/v) PEG 2000 MME, 0.1 M Tris, pH 7.0 | rectangle |
| 11.7[2] | 10% (v/v) 2-propanol, 0.1 M MES pH 6.0, 0.2 M Ca $(OAc)_2$ 7.0, 0.2 M NaCl | 6-sided crystal |
| 11.7[2] | 30% (w/v) PEG-3000, 0.1 M Tris pH | cube |
| 11.7[2] | 20% (w/v) PEG-3000, 0.1 M Tris pH 7.0, 0.2 M Ca $(OAc)_2$ imidazole pH 8.0, 0.2 M $Zn(OAc)_2$ | 6-sided crystal |
| 11.7[2] | 20% (w/v) PEG-3000, 0.1 M imidazole, pH 8.00 | oval |
| 11.7[2] | 20% (w/v) PEG-3000, 0.1 M imidazole pH 8.0, 0.2 M $Zn(OAc)_2$ | 4- sided diamond |
| 11.7[2] | 30% (v/v) PEG-600, 0.1 M MES, pH 6.0, 5% (w/v) PEG-1000, 10% (v/v) glycerol | cube |
| 11.7[2] | 40% (v/v) PEG-600, 0.1 M phosphate-citrate pH 4.2 6.0, 5% (w/v) PEG-3000 | cube |
| 11.7[2] | 40% (v/v) PEG-400, 0.1 M MES pH | Cube |
| 11.7[2] | 40% (v/v) PEG-6000, 0. M CHES pH 9.5 | Cube |
| 11.7[2] | 40% (v/v) PEG-6000, 0.1 M CHES pH. 9.5 | oval |
| 11.7[2] | 40% (v/v) PEG-400, 0.1 M acetate pH 4.5 | cube |
| 17[2] | 30% (v/v) PEG 600, 0.1 M MES, pH 6.00, 0.2 M NaCl | diamond |
| 17[2] | 40% (v/v) PEG 300, 0.1 M phosphate-citrate, pH 4.2. | Cube |
| 17[2] | 50% (v/v) PEG 200, 0.1 M phosphate-citrate, pH 4.2., 0.2 M NaCl | Needle stack |
| 17[2] | 40% (v/v) PEG 400, 0.1 M Tris, pH 8.50, 0.2 M $Li_2SO_4$ | Rods |
| 17[2] | 30% (v/v) PEG 600, 0.1 M MES, pH 6.00, 5% (w/v) PEG 1000, 10% glycerol | Diamond |
| 17[2] | 40% (v/v) PEG 400, 0.1 M citrate, pH 5.5, 0.2 M NaCl | Cube |
| 17[2] | 40% (v/v) PEG 400, 0. M Na/phosphate, pH 6.2, 0.1 M NaCl | 6-sided crystal |
| 17[2] | 40% (v/v) PEG 600, 0.1 M CHES, pH 9.50 | cube |
| 17[2] | 50% (v/v) MPD, 0.1 M cacodylate, pH 6.50, 5% (w/v) PEG 8000 | oval |
| 17[2] | 40% (v/v) PEG 400, 0.1 M HEPES, pH 7.50, 0.2 M Ca $(Ac)_2$ | 6-sided crystal |
| 17[2] | 40% (v/v) PEG 300, 0.1 M CHES, pH9.50, 0.2 M sodium citrate | Uncomplete rods |
| 17[2] | 50% (v/v) PEG 200, 0.1 M phosphate-citrate, pH 4.20, 0.2 MNaCl | Uncomplete rods + oval |
| 17[2] | 10% (w/v) PEG 8000, 0.1 M MES, pH 6.00, 0.2 M Zn $(OAc)_2$ | Needle haystack |
| 17[2] | 40% (v/v) PEG 400, 0.1 M Tris, pH 8.50, 0.2 M $Ca(OAc)_2$ | dendritic |

(continued)

| OXO Concentration (mg/ml) | Precipitant | Description of Crystals |
|---|---|---|
| 17[2] | 40% (v/v) PEG 600, 0.1 M imidazole, pH 8.00, 0.2 M $Zn(OAc)_2$ | rods |
| 1. 50 mM succinate buffer, pH 4.50<br>2. 50 mM succinate buffer, 150 mM $(NH_4)_2SO_4$, pH 4.5 | | |

## Example 5: Crystallization of OXO (Microbatch)

Oxalate oxidase could be crystallized by the microbatch method from a number of crystallization conditions:

(a) 20 µl of purified oxalate oxidase at a concentration of 27 mg/ml by A 280 nm was mixed with 10 µl of a mixture composed of 40% PEG, 600 in 0.1 M phosphate-citrate, pH 4.2. A further 10 µl of PEG 600 was added and mixed. Finally, 5 µl of glycerol was added and thoroughly mixed. Crystallization occurred within 10 minutes. The yield was 75%. There was no precipitation.
(b) 100 µl of oxalate oxidase (27 mg/ml) was mixed with 55 µl of a solution composed of 40% PEG, 600 in 0.1 M phosphate-citrate, pH 4.2. An additional 25 µl of PEG 600 was added and mixed thoroughly.
(c) 100 µl oxalate oxidase (27 mg/ml) was mixed with 50 µl of a solution composed of 40% PEG 600 in 0.1 M phosphate-citrate, pH 4.2. An additional 30 µl of PEG 600 was added and mixed thoroughly. Oxalate oxidase crystal cubes developed within 2 hours.
(d) Oxalate oxidase (27 mg/ml) was mixed with 20 µl of a solution composed of 40% (v/v) PEG 300, 0.1 M citrate, pH 4.5.
(e) 66.6 µl of oxalate oxidase at a concentration of 9.34 mg/ml was mixed with 133.4 µl of a solution composed of 40% (v/v) PEG 400, 0.1 M MES, pH 6.00, and 5% (w/v) PEG 3000.
(f) To 23.25 µl of oxalate oxidase (43 mg/ml), 1.65 µl of 1M Tris, pH 7.00 was added and mixed followed by stepwise addition and mixing of 4.15 µl of 2 M $(NH_4)_2SO_4$, 1.5 µl of 100% glycerol, 12 µl of 100% PEG 600, 7.45 µl of water. Final concentrations were: 20 mg/ml oxalate oxidase, 166 mM $(NH_4)_2SO_4$, 0.33 M Tris, pH 7.00, 3% glycerol, 24% PEG 600, 20 mg/ml oxalate oxidase.

## Example 6: Crystallization of OXO (Batch)

*Batch Crystallization:* Oxalate oxidase could be crystallized by the batch method from a number of crystallization buffers:

(a) The crystallization condition was a mixture of 13.9 mg/ml oxalate oxidase, 65 mM citrate-phosphate buffer, pH 3.50 and 28.5% PEG 600. Crystals were cubes less than 5 µm in size.
(b) To 1.19 µl oxalate oxidase (43 mg/ml) was added in a stepwise fashion, 0.35 µl 100% PEG 600, 0.12 µl of 2 M $(NN_4)_2SO_4$, 0.20 µl 1M Tris, pH 7.00, and 0.26 µl of water. Cube shaped crystals appeared within an hour.

*Large Scale Crystallization:* Crystallization was scaled up to 15 ml batches to supply material for pre-clinical studies.

(a) 10 ml of purified oxalate oxidase at a concentration of 21.5 mg/ml was mixed with 2 ml of 0.5 M citrate-phosphate buffer, pH 3.50. 4.7 ml 100% PEG 600 was added and thoroughly mixed. The crystallizing solution was allowed to tumble overnight at room temperature. The yield was 94%.
(b) Purified OXO was mixed with crystallization buffer in 1:2 ratio, buffer containing 40% PEG 400, 0.1 M MES, pH 6, 5% PEG 300, and tumbled at room temperature overnight. This procedure produces diamond-shaped OXO crystals in high (> 70%) yield.
(c) 21.5 mg/ml OXO in 65 mM citrate phosphate buffer pH 3.5 was mixed with 28.5% PEG 600 and left at room temperature overnight.

## Example 7A: Cross-linking of OXO Crystals

Oxalate oxidase crystals [from where?] were cross-linked using glutaraldehyde. After crystallization, oxalate oxidase crystals were concentrated to 20-30 mg/ml. 3.2 ml of 25% glutaraldehyde was added to 20 ml of crystals to make a solution of 4% glutaraldehyde, and crystals tumbled for 16 hours at room temperature. Cross-linked crystals

were washed five times with 100 mM Tris, pH 7.00 and resuspended in 10 mM Tris, pH 7.00.

Specific activities of soluble oxalate oxidase and CLEC oxalate oxidase were compared (six trials) and it was shown that cross-linked oxalate oxidase crystals retain more than 70% to more than 95% of the original activity of the soluble protein, in various preparations.

**Example 7B: Crosslinking of Oxalate Oxidase Crystals**

Oxalate oxidase crystals, prepared as described in Example 6 (large scale), were crosslinked by addition of glutaraldehyde (Sigma). A 1 ml aliquot of oxalate oxidase crystals(60 mg/ml) was crosslinked with different concentrations of glutaraldehyde (from 0.05% to 2%, final concentration)at pH 4.2 at 25°C for 12 hrs. The crosslinking was terminated by separation of the crosslinked crystals by centrifugation at 2000 rpm in an eppendorf and resuspending the crosslinked crystals in 1 ml of 100 mM Tris.HCl, pH 7.0. The CLEC was then washed five times with 100 mM Tris.HCl buffer, pH 7.5 followed by three times with 10 mM Tris.HCl buffer, pH 7.5.

**Example 7C: pH Controlled Solubility of Crosslinked Oxalate Oxidase Crosslinked Crystals**

Solubility of various crosslinked oxalate oxidase crystals was studied following a decrease in pH from 7.5 to 3.0. The crosslinked crystals were incubated at 1 mg/ml in 50 mM glycine.HCl (pH 3). Aliquots were removed after 5 hour incubation at 37°C with stirring. Soluble protein concentration was measured at OD 280 nm after separation of the undissolved crosslinked crystals by centrifugation at 2000 rpm and filtration of the supernatant through 0.22 u filter. The results are described in Table 5 below.

Table 5: OXO-CLEC pH Stability

| Sample | Glutaraldehyde (%) | Protein Leaching (%) |
|---|---|---|
| OXO-CLEC-1 | 0.05 | 100.0 |
| OXO-CLEC-2 | 0.10 | 2.9 |
| OXO-CLEC-3 | 0.25 | 2.3 |
| OXO-CLEC-4 | 0.50 | 0.0 |
| OXO-CLEC-5 | 1.00 | 0.0 |
| OXO-CLEC-6 | 2.00 | 0.0 |

Together with the results of Example 7A, this shows that a substantially stable glutaraldehyde oxalate oxidase crosslinked crystal is formed in the presence of at least about 0.1 % (final concentration) glutaraldehyde. Preferably, at least 0.5% final concentration, and more preferably 4% is used.

**Example 7D: Stability of Crosslinked Oxalate Oxidase Crystals at Various pHs**

Oxalate oxidase crystals, prepared as described in Example 7B, were crosslinked by addition of glutaraldehyde (Sigma). A 1 ml aliquot of oxalate oxidase crystals (60 mg/ml) was crosslinked with 1% glutaraldehyde (final concentration) at pH 4.2 at 25°C for 12 hrs. The crosslinking was terminated by separation of the crosslinked crystals by centrifugation at 2000 rpm in an eppendorf and resuspending the crosslinked crystals in 1 ml of 100 mM Tris.HCl, pH 7.0. The CLEC was then washed five times with 100 mM Tris.HCl buffer, pH 7.5 followed by three times with 10 mM Tris.HCl buffer, pH 7.5.

Stability of crosslinked oxalate oxidase crystals was studied by incubating 20mg/ml of the crosslinked crystals of oxalate oxidase at 37°C at two different pHs 2.0 (50 mM glycine.HCl buffer) and 7.00 (50mM Tris.HCl buffer). Aliquots were removed at different intervals of time and the activities were measured in pH 3.8 as described in Example 11. The results are described in Table 6.

Table 6: OXO-CLEC pH Stability

| | Activity (%)* | | |
|---|---|---|---|
| | 0 hr | 2 hr | 5 hr |
| pH 2.0 | 100 | 119 | 109 |

(continued)

| | Activity (%)* | | |
|---|---|---|---|
| | 0 hr | 2 hr | 5 hr |
| pH 7.0 | 100 | 96 | 104 |
| * Results above 100% are within experimental error. What these results show is that crosslinked oxalate crystals are stable at various pHs. | | | |

**Example 7E: pH Activity Profile of Crosslinked Oxalate Oxidase Crystals**

Oxalate oxidase crystals, prepared as described in Example 7B, were crosslinked by addition of glutaraldehyde (Sigma). A 1 ml aliquot of oxalate oxidase crystals(60 mg/ml) was crosslinked with 4% glutaraldehyde (final concentration) at pH 4.2 at 25°C for 12 hrs. The crosslinking was terminated by separation of the crosslinked crystals by centrifugation at 2000 rpm in an eppendorf and resuspending the crosslinked crystals in 1 ml of 100 mM Tris.HCl, pH 7.0. The CLEC was then washed five times with 100 mM Tris.HCl buffer, pH 7.5 followed by three times with 10 mM Tris.HCl buffer, pH 7.5. The pH activity profile of crosslinked oxalate oxidase crystals was studied by measuring the activity of crosslinked crystals of oxalate oxidase as described in Example 11 using various buffers and pHs: 50 mM glycine.HCl buffer at pH 2.0 and 3.0, 50 mM succinate buffer at pHs 4.0, 5.0, 6.0 and 50 mM Tris buffer at pH 7.0. The results are shown in the Figure 11. For comparative purposes soluble oxalate oxidase is also shown.

**Example 8: OXO Therapy in Animal Model For Type I Primary Hyperoxaluria**

*Mouse Model For Type I Primary Hyperoxaluria:* AGT1 knockout mice lack the liver peroxisomal enzyme alanine:glyoxylate aminotransferase, a deficiency in which results in type I primary hyperoxaluria. These knockout mice exhibit mild hyperoxaluria. AGT1 KO mice have urinary oxalate levels that are elevated approximately 5-fold relative to wild type mice (their daily excretion is approximately 1-2 mmol/L, as compared to normal urinary levels of 0.2 mmol/L).

A total of 37 male mice (strain AGT1 KO/C57BL6, developed by Dr. Salido, La Laguna Tenerife, Spain) were used in these experiments. Mice were randomly divided between a control group and two experimental groups. Mice weighed 20-25 grams and were less than 6 months of age.

*Administration of OXO crystals:* Mice were acclimated prior to treatment for 7 days to individual metabolic cages (Tecniplast USA Inc, Exton, PA, USA), and were fed standard breeder diet (17% proteins, 11% fat, 53.5% carbohydrate) containing less than 0.02-0.08% oxalate and less than 0.5% calcium.

For the duration of the 12-day treatment period, each mouse was administered a treatment by gavage twice daily, at 10 a.m. and 5 p.m., with a curved stainless steel 18 gauge feeding needle (Harvard Apparatus Inc.; Holliston, MA). Mice in the control group received 1.2 ml saline two times a day by gavage; mice in the experimental groups received 50 mg of oxalate oxidase per day, either as the soluble enzyme or as a suspension of cross-linked oxalate oxidase crystals by gavage (1.2 ml volume, twice daily). Animals in all experimental groups showed slight adverse effect to gavage. Soluble oxalate oxidase was administered in 50 mM citrate-phosphate buffer pH 6.2. Cross-linked oxalate oxidase crystals were administered as a suspension in 10 mM Tris.HCl buffer (pH 7.0).

*Analysis of urine samples:* 24 h urinary samples were collected in metabolic cages over acid (15 μl of 1N hydrochloride acid per 3-4 ml of urine) in order to minimize the spontaneous breakdown of urinary ascorbic acid to oxalate. Samples were stored at -70°C until further analysis. Daily diuresis, oxalate and creatinine excretion were measured. Assays for oxalate and creatinine are described in Example 11. Urinary excretion of oxalate was expressed as a molar ratio of urinary excretion of creatinine. Data were analyzed statistically using Student's t-test.

As shown in Figure 8, no effect on urinary oxalate was measured with the administration of soluble OXO, whereas treatment with cross-linked OXO crystals resulted in a statistically significant reduction in urinary oxalate levels after 12 days of treatment.

**Example 9: OXO Therapy in Animal Model For Enteric Hyperoxaluria**

*Rat Model For Enteric Hyperoxaluria:* Male Prague Dawley rats fed a diet high in oxalate constitute a suitable animal system for the study of enteric hyperoxaluria. Administration of 1.5% dietary ammonium oxalate, in conjunction with antibiotic treatment to eliminate the enteric oxaiate-degrading enteric bacterium *Oxalobacter formigenes,* resulted

in a 5- to 10-fold increase in urinary oxalate in this study.

Sprague Dawley rats less than 35 days old and weighing 100-120 grams were randomly divided into a control group and experimental groups (six rats per group, with six rats in a spare group). Rats were acclimated for 7 days to individual metabolic cages (LabProducts, Inc.; Seaford, DE) prior to treatment. During this period, rats were provided *ad libitum* with acidified water supplemented with the tetracycline antibiotic Terramycin™ (500mg/L) to eliminate *Oxalobacter formigenes,* and fed a synthetic diet having 1.5% ammonium oxalate and a low (0.5%) concentration of calcium (Research Diets TD89222PWD; Harlen Teklad; Madison, WI). Rats were maintained on this diet for the duration of the treatment. Antibiotic treatment was discontinued after the initial seven day acclimatization period.

OXO was administered by gavage three times daily (at 9 a.m., 12 p.m., and 4 p.m.), using 18 gauge gavage needles (Harvard Apparatus Inc.; Holliston, MA) for the duration of the 12-day treatment period. The gavage control group received 1 ml water. Rats in the experimental group received a 1 ml suspension of cross-linked (4% glutaraldehyde, *see, e.g.,* Example 7) oxalate oxidase crystals by gavage (15 mg/rat/day) in 10 mM Tris-HCl buffer (pH 7.0).

*Analysis of urine samples:* 24 hour urinary samples were collected in metabolic cages over acid (50 μl of 1N hydrochloride acid per 10 ml of urine sample) in order to minimize the spontaneous breakdown of urinary ascorbic acid to oxalate. Samples were stored at -70°C until further analysis. Daily diuresis, oxalate and creatinine excretion were measured. Assays for oxalate and creatinine are described in Example 11. Urinary excretion of oxalate was expressed as a molar ratio of urinary excretion of creatinine. Data were analyzed statistically using Student's t-test.

As shown in Figure 9, as of day 7, administration of cross-linked oxalate oxidase crystals resulted in a significant and sustained decrease in urinary oxalate excretion, and up to a 40% reduction in urinary oxalate levels after 12 days of treatment.

## Example 10: OXO Therapy For the Treatment of Oxalate-Related Disorders in Humans

Humans in need for treatment or prevention of an oxalate-related disorder are treated by administration of oxalate oxidase crystals orally. The oxalate oxidase crystals are administered as a dose of approximately 10 μg/kg to 25 mg/kg, 1 mg/kg to 25 mg/kg, or 5 mg/kg to 100 mg/kg, as determined by a treating clinician, depending on the severity of the symptoms and the progression of the disease. The oxalate oxidase crystals, crosslinked using glutaraldehyde, are administered 1, 2, 3, 4, or 5 times daily, or are administered less frequently, such as once or twice a week. Treatment with oxalate oxidase crystals results in a decrease in urinary oxalate levels of at least 10%, 20%, 25%, 30%, 35%, 40%, 45%, 50%, 55%, 60%, 70% or more.

## Example 11: Assays

*Protein Concentration Determination:* The concentration of oxalate oxidase was determine by measuring absorbance at 280 nm. The absorbance of 1 optical density (OC) was considered as 1 mg/ml.

*OXY Activity Assay:* The following Sigma Aldrich protocol (Enzymatic Assay of Oxalate Oxidase EC 1.2.3.4) was used to measure the activity of oxalate oxidase (OXO). A unit of enzymatic activity is defined as follows: one Unit will form 1.0 μmol of $H_2O_2$ from oxalate per minute at pH 3.8 at room temperature.

Assay samples were normalized at a concentration of 0.25 mg/mL in 50 mM succinate buffer solution containing 0.79 mM N,N-dirriethylaniline (DMA) (Sigma), 0.11 mM 2-methyl-2-benzothiazolinone hydrazone hydrochloride (MBTH) (Sigma), pH 3.8 and kept on ice; protein concentration was determined by absorbance at 280 nm. A 1 mg/mL peroxidase solution was prepared in cold di$H_2$O prior to use and kept on ice. All other reagents were kept at room temperature. The assay was performed at room temperature. As the first reaction is $O_2$-dependent, it is important to oxygenate master mix before addition of oxalate oxidase. Reagents were added to a 50 ml tube in the following order: 27 mL 50 mM succinate buffer adjusted to pH to 3.8 using 1M NaOH; 1 mL 100 mM EDTA; and 1 ml distilled water, and strongly oxygenated for 30 minutes. First, 2.9 ml of the oxygenated working solution was transferred to a 3 ml plastic cuvette and mixed well with 20 μl of 200 mM Oxalic Acid Solution, pH 3.8 and 10 μl of 1 mg/mL peroxidase. Then 10 μl of oxalate oxidase were added and mixed quickly by inverting 3 times in a closed tube. Changes in absorbance at 600 nm were immediately read and recorded for up to 3 minutes over 10 second time intervals using a Shimadzu Biospec. The linear portion of curve (between 0 and 120 seconds) was used to estimate the change in absorbance at 600 nm per minute, for both the test and the blank. The sample blank was comprised of all assay reagents except oxalate oxidase.

Enzyme specificity was calculated as follows,

$$U/mL\ enzyme = \{(\Delta\ A600/min)(2.95)(dilution\ factor)\} / \{(26.4)(0.02)\}$$

and

$$U/mg\ solid= \{U/mL\ enzyme\} / \{mg\ solid/ mL\ enzyme\}$$

where 2.95 = total volume in milliliters of assay, df = dilution factor, 26.4 = millimolar extinction coefficient of indamine dye at 600 nm, and 0.02 = Volume of enzyme used in milliliters.

In a specific experiment, cross-linked crystals of oxalate oxidase (CLEC OXO) (diamond shaped crystals cross-linked with 4% glutaraldehyde by tumbling overnight) were compared to soluble OXO. As shown in Figure 4, CLEC OXO retains 95.5% of the activity of the corresponding soluble OXO preparation.

*Oxalate determination by colorimetric method:* Oxalate colorimetric kit for quantitative determination of oxalate in the urine were purchased from Trinity Biotech USA (St Louis, MO) or Greiner Diagnostic AG (Dennliweg 9, Switzerland). The urine samples were diluted and treated according to the manufacturer's instruction. The assay comprises two enzymatic reactions: (a) oxalate is oxidized to carbon dioxide and hydrogen peroxide by oxalate oxidase, and (b) the hydrogen peroxide thus formed reacts with 3-methyl-2-benzothiazolinone hydrazone (MBTH) and 3-(dimethylamino) benzoic acid (DMAB) in the presence of peroxidase to yield an indamine dye which can be detected by absorbance at 590 nm. The intensity of the color produced is directly proportional to the concentration of oxalate in the sample. Urine oxalate values are calculated from standard curve.

*Creatinine determination by colorimetric method:* Creatinine colorimetric kits for the quantitative determination of creatinine in the urine were purchased from Quidel Corporation (San Diego, CA; METRA Creatinine Assay kit) or Randox Laboratories (Antrim, United Kingdom). The assay is based on the principle that creatinine reacts with picric acid in alkaline solution to form a product that has an absorbance at 492 nm. The amount of complex formed is directly proportional to the creatinine concentration. 24 h rat urine samples collected from single metabolic cages were diluted 15-fold with double distilled water. 20 μl of diluted urine sample was mixed with 20 μl picric acid/sodium hydroxide (1: 1). Absorbance at 492 nm was measured after incubating for 2 minutes incubation at room temperature. Urinary creatinine values were calculated from standard curve.

*Periodic Acid Schiff Staining:* Soluble and crystalline oxalate oxidase were separated on a 4-20% tris-glycine gel. At the end of the run, the gel was cut into two halves. The left half (lane 1-5) was stained with Coomassie blue stain and the right half (lanes 7-10) was stained with PAS stain. For PAS staining, the gel was washed four times, 10 min per wash, with 40% methanol and 7% acetic acid. The washed gel was shaken in fresh 40% methanol, 7% acetic acid solution overnight at room temperature. After washing a few times with deionized water, the gel was incubated in a solution containing 1% periodic acid and 3% acetic acid for 1 hour with shaking. The final incubation with Schiff reagent was carried out for 1 hour in the dark after extensive washed with DI water (Figure 2).

## Example 12: OXO Therapy in Animal Model for Primary Hyperoxaluria

The AGT1 KO (C57B16) mice (phenotype described in Example 8) were challenged with ethylene glycol (EG) to provoke severe hyperoxaluria. EG is common alcohol that is metabolized in the liver to oxalate.

Mice were acclimated prior to treatment for 7 days to individual metabolic cages (Tecniplast USA Inc, Exton, PA, USA), and were fed standard breeder diet (17% proteins, 11% fat, 53.5% carbohydrate) containing less than 0.02-0.08% oxalate and approximately 0.5-0.9% calcium. After an acclimation period, mice were given water supplemented with 0.7% EG *ad libitum* for 7 days and the same was continued during the study. After several days of challenge, the mice were excreting approximately 10-20-fold more oxalate in their urine as compared to wild type mice with the daily excretion being in the range of 3-6 mmol/L.

*Administration of OXO-CLEC enzyme:* A total of 8 male mice strain AGT1 KO/C57 B16 were pre-challenged for 7 days with EG and then a single dose of recombinant oxalate oxidase formulated as cross linked crystals (4% glutaraldehyde *see, e.g.,* Example 7) was given for 11 consecutive days orally as a freeze/dried food enzyme mixture (50 mg of enzyme in a suspension of 10MM Tris.HCl buffer (pH7) was mixed with 5 gm food, freeze dried and each morning food containers were re-filled with ~7gm of food/enzyme mixture) ("dose 50"). Mice were randomly divided between a control group and experimental group. Mice weighed 20-25 grams and were less than 6 months of age.

*Analysis of urine samples:* 24 h urinary samples were collected in metabolic cages over acid (15 μl of 1N hydrochloride acid per 3-4 ml of urine) in order to minimize the spontaneous breakdown of urinary ascorbic acid to oxalate. Samples were stored at -20 °C until further analysis. Daily diuresis, oxalate and creatinine excretion were measured. Assays for oxalate and creatinine are described in Example 10. Urinary excretion of oxalate was expressed as μmol of oxalate excreted in 24 h urine sample (mL). Data were analyzed statistically using Student's t-test. *See* Figure 12

As shown in Figure 12, oral administration of OXO-CLEC to EG AGT1KO (C57B16) mice resulted in significant

reduction of urinary oxalate levels from day 6 of the treatment until the end of the study when compared with matched untreated control mice.

**Example 13: OXO Therapy in Animal Model for Severe Hyperoxaluria, Young EG AGT1KO (129/sv) Mice**

The AGT1 KO (129/sv) mice were challenged with ethylene glycol (EG) to provoke severe hyperoxaluria and formation of calcium oxalate deposits in the kidney parenchyma. Usually 2-6 weeks after challenge, the mice show signs of impaired kidney function judged by variable excretion of oxalate in the urine, variable degree of nephrocalcinosis and decreased creatinine clearance.

Mice were acclimated prior to treatment for 7 days to individual metabolic cages (Tecniplast USA Inc, Exton, PA, USA), and were fed standard breeder diet (17% proteins, 11% fat, 53.5% carbohydrate) containing less than 0.02-0.08% oxalate and less than 0.5-0.0.9% calcium).

*Administration of OXD-CLEC enzyme:* A total of 22 male mice strain AGT1 KO/ 129/sv were pre-challenged for 7 days with EG. During this period mice were provided *ad libitum* with 0.7% ethylene glycol water to create sever hyperoxaluria. After 7 days of pre-treatment, a single dose of recombinant oxalate oxidase formulated (50 mg OXO enzyme suspended in 10mM Tris.HCl buffer (pH7) as cross linked crystals (4% glutaraldehyde see, e.g., Example 7) was given for 31 consecutive days orally as a freeze/dried food enzyme mixture (50 mg of the enzyme suspended as described above was mixed with 3.5 gm food, freeze dried and each morning food containers were re-filled with ~7gm of food/enzyme mixture). Mice were randomly divided between a control group and experimental group. Mice weighed ~20-25 grams and were less than 8-10 weeks of age.

Analysis *of urine samples:* 24 h urinary samples were collected in metabolic cages over acid (15 μl of 1N hydrochloride acid per 3-4 ml of urine) in order to minimize the spontaneous breakdown of urinary ascorbic acid to oxalate. Samples were stored at -20 °C until further analysis. Daily diuresis was recorded and concentration of urinary oxalate and creatinine was measured two times a week. Assays for oxalate is described in Example 11. Urinary excretion of oxalate was expressed as μmol of oxalate excreted in 24 h urine sample (mL). Data were analyzed statistically using Student's t-test. *See* Figure 13.

As shown in Figure 13, oral administration of OXO-CLEC to EG AGT1KO (129/sv) mice resulted in reduction of urinary oxalate levels from day 3 of the treatment when compared with matched untreated control mice, reduction was maximal and significant after 10 days of treatment.

*Assessment of the renal function by creatinine clearance measurement.* At the end of the study all animals that survived 4 weeks of EG challenge were sacrificed and blood was collected to measure plasma creatinine and creatinine clearance. For serum creatinine measurement a slightly modified Jaffe reaction method by Slot. C, 1965 and Heinegard D, 1973, kit Oxford Medical Research) were used. 80 ul of undiluted serum samples were mixed with 800 ul of picric alkaline in the cuvettes and incubated for 30 minutes at room temperature. Color development was measured spectroptometrically at 510 nm; at that point 33.3 μL of 60% acetic acid was added to quench unspecific reactions. Samples were thoroughly mixed and after 5 minutes incubation at room temperature were read again at 510 nm: Final absorbance is presented as a difference of two readings. Serial dilution of 1 mM creatinine solution was used for the Standard curve.

Creatinine clearance is expressed as excretion rate ($U_{cr}$ x V), where $U_{cr}$ represents the concentration of creatinine (($\mu$mol/L) in a urine sample, divided by plasma creatinine ($P_{cr}$). This is represented as:

$$C_{cr} = (U_{cr} x V) / P_{cr} = mL/h$$

As shown in Figure 14, 4 weeks of treatment with OXO-CLEC maintained normal kidney function expressed by creatinine clearance (7/11), while in the control group only 2 mice (2/11) survived EG challenge and their kidney filtration rate was below normal range (4.76 ml/h)

*Kidney histoptahology analysis:* Mouse kidneys were routinely processed for paraffin embeding and positioned in order to obtain complete cross sections of the kidneys. Each kidney was cut in 12 serial sections at 4 μm per kidney and stained with either hemotoxylin and eosin for routine histological examination, or by specific Yasue metal substitution histochemical method to detect the presence of calcium oxalate crystals in the renal tissue. Slides were examined under the microscope using 20X magnification and examiner scored sections under 4-category scale, applying the same criteria to each of the anatomic areas in the kidney (cortex, medulla and papilla). The scoring was none, no oxalate crystals in any field, minimal, 1-5 crystals in any field, moderate 5-10 crystals in any field, and severe, all fields with multiple collections of crystals. See Figure 15 and Table 6.

Table 6: Severity of Nephrocalcinosis and Number of Mice Affected in Treated vs Control Group

| Groups | Severe | Moderate | Minimal | None | CaOx Deposits (%) |
|---|---|---|---|---|---|
| CONT n=11 | 9 (died) | 1 | 1 | - | 100 |
| 50 mg n=11 | 4 (died) | 2 | - | 5 | 54.5 |
| * All treatment groups and matched controls had n=11 mice at the beginning of the study. At the end of the study, all animals that survived were sacrificed and histopatological analysis was performed. Mice that died during the study or were sacrificed due to sickness were also examined. | | | | | |

As shown in Table 6 and Figure 15, four weeks of oral treatment with oxalate decarboxylase-CLEC prevented formation of calcium oxalate deposits in kidney parenchyma of AGT1KO mice that were challenged with ethylene glycol. 5 mice from the treatment group (5/11) had absolutely normal kidney morphology with no traces of calcium oxalate crystals, while 100% of mice from untreated control group had CaOx deposits (11/11).

*Survival rate analysis by Kaplan-Meier estimator:* Effect of OXO-CLEC treatment on survival rate of mice challenged with ethylene glycol was analyzed using Kaplan-Meier method where survival of subjects that died in the certain time point is divided by the number of subjects who were still in the study at the time. This method is simple and graphically illustrates difference between two or more groups that were in the study. Often statistical programs such as Kaleida graph, STATS are used for calculations.

As depicted in Figure 16, 4 weeks of oral treatment with oxalate oxidase-CLEC significantly increased survival rate of ethylene glycol challenged AGT1K0 mice. Notice that estimated median survival time (the time that at which 50% of the mice from the treatment group survived) was 31 days, that is actual duration of the study.

The specification is most thoroughly understood in light of the teachings of the references cited within the specification. The embodiments within the specification provide an illustration of embodiments of the invention and should not be construed to limit the scope of the invention. The skilled artisan readily recognizes that many other embodiments are encompassed by the invention.

Unless otherwise indicated, all numbers expressing quantities of ingredients, cell culture, treatment conditions, and so forth used in the specification, including claims, are to be understood as being modified in all instances by the term "about." Accordingly, unless otherwise indicated to the contrary, the numerical parameters are approximations and may vary depending upon the desired properties sought to be obtained by the present invention. Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein.

## Claims

1. Use of oxalate oxidase crystals in the manufacture of a medicament for the treatment of a disorder associated with elevated oxalate concentration in a mammal, wherein the oxalate oxidase crystals are stabilized and comprise oxalate oxidase covalently linked by a cross-linking agent, wherein the medicament is for oral administration.

2. The use of claim 1, wherein the disorder is selected from the group consisting of a kidney disorder, bone disorder, liver disorder, gastrointestinal disorder, and pancreatic disorder.

3. The use of claim 1, wherein the disorder is selected from the group consisting of primary hyperoxaluria, enteric hyperoxaluria, idiopathic hyperoxaluria, ethylene glycol poisoning, cystic fibrosis, inflammatory bowel disease, urolithiasis, and nephrolithiasis.

4. The use of claim 1, wherein the cross-linking agent is multifunctional.

5. The use of claim 1, wherein the cross-linking agent is bifunctional.

6. The use of claim 5, wherein the bifunctional cross-linking agent is glutaraldehyde.

7. The use of claim 1, wherein the oxalate oxidase crystals are to be administered as a suspension, dry powder, capsule, or tablet.

8. The use of claim 1, wherein administration of oxalate oxidase crystals results in a reduction of oxalate concentration of at least 10%.

9. The use of claim 8, wherein administration of oxalate oxidase crystals results in a reduction of oxalate concentration of at least 20%.

10. The use of claim 9, wherein administration of oxalate oxidase crystals results in a reduction of oxalate concentration of at least 30%.

11. The use of claim 8, 9, or 10, wherein the reduction is measured in a biological sample selected from the group consisting of urine, blood, plasma, and serum.

12. The use of claim 1, wherein the oxalate oxidase is recombinantly produced.

13. An oxalate oxidase crystal cross-linked with a multifunctional cross-linking agent for use in the treatment of an oxalate-related disorder by oral administration.

14. The cross-linked oxalate oxidase crystal for use according to claim 13 where the cross-linking agent is glutaraldehyde.

15. The cross-linked oxalate oxidase crystal for use according to claim 13 where the cross-linking agent is glutaraldehyde and is present in a final concentration of at least about 0.1 %.

16. The cross-linked oxalate oxidase crystal for use according tu claim 13 where the cross-linking agent is glutaraldehyde and is present in a final concentration of 4%.

17. The cross-linked oxalate oxidase crystal for use according to claim 13 where the cross-linking agent is glutaraldehyde and is present in a final concentration of 1%.

18. The crystal for use according to anyone of claims 13 to 17, wherein the oxalate oxidase retains at least 70% of the activity of the corresponding soluble oxalate oxidase.

19. The crystal for use according to anyone of claims 13 to 17, wherein the oxalate oxidase retains at least 95% of the activity of the corresponding soluble oxalate oxidase.

20. The crystal for use according to anyone of claims 13 to 17, wherein the oxalate oxidase retains at least 98% of the activity of the corresponding soluble oxalate oxidase.

21. A pharmaceutical composition for use in the treatment of an oxalate-related disorder by oral administration comprising the crystal of anyone of claims 13 to 17.

**Patentansprüche**

1. Verwendung von Oxalat-Oxidase-Kristallen bei der Herstellung eines Medikaments für die Behandlung einer Funktionsstörung, die mit einer erhöhten Oxalatkonzentration in einem Säugetier einhergeht, wobei die Oxalat-Oxidase-Kristalle stabilisiert sind und Oxalat-Oxidase umfassen, die über ein Vernetzungsmittel kovalent gebunden ist, wobei das Medikament zur oralen Verabreichung ist.

2. Verwendung nach Anspruch 1, wobei die Funktionsstörung ausgewählt ist aus der Gruppe bestehend aus einer Nierenfunktionsstörung, Knochenfunktionsstörung, Leberfunktionsstörung, Magen- und Darmfunktionsstörung und Bauchspeicheldrüsenfunktionsstörung.

3. Verwendung nach Anspruch 1, wobei die Funktionsstörung ausgewählt ist aus der Gruppe bestehend aus primärer Hyperoxalurie, enterischer Hyperoxalurie, idiopathischer Hyperoxalurie, Ethylenglycol-Vergiftung, zystischer Fibrose, inflammatorischer Darmerkrankung, Urolithiasis und Nephrolithiasis.

4. Verwendung nach Anspruch 1, wobei das Vernetzungsmittel multifunktional ist.

5. Verwendung nach Anspruch 1, wobei das Vernetzungsmittel bifunktional ist.

6. Verwendung nach Anspruch 5, wobei das bifunktionale Vernetzungsmittel Glutaraldehyd ist.

7. Verwendung nach Anspruch 1, wobei die Oxalat-Oxidase-Kristaile als Suspension, Trockenpulver, Kapsel oder Tablette zu verabreichen sind.

8. Verwendung nach Anspruch 1, wobei Verabreichung der Oxalat-Oxidase-Kristalle zu einer Reduktion der Oxalatkonzentration von wenigstens 10% führt.

9. Verwendung nach Anspruch 8, wobei Verabreichung der Oxalat-Oxidase-Kristalle zu einer Reduktion der Oxalatkonzentration von wenigstens 20% führt.

10. Verwendung nach Anspruch 9, wobei Verabreichung der Oxalat-Oxidase-Kristalle zu einer Reduktion der Oxalatkonzentration von wenigstens 30% führt.

11. Verwendung nach Anspruch 8, 9 oder 10, wobei die Reduktion in einer biologischen Probe gemessen wird, ausgewählt aus der Gruppe bestehend aus Urin, Blut, Plasma und Serum.

12. Verwendung nach Anspruch 1, wobei die Oxalat-Oxidase rekombinant hergestellt wird.

13. Ein Oxalat-Oxidase-Kristall vernetzt mit einem multifunktionalen Vernetzungsmittel zur Verwendung bei der Behandlung einer Oxalat-betreffenden Funktionsstörung durch orale Verabreichung.

14. Das vernetzte Oxalat-Oxidase-Kristall zur Verwendung nach Anspruch 13, wobei das Vernetzungsmittel Glutaraldehyd ist.

15. Das vernetzte Oxalat-Oxidase-Kristall zur Verwendung nach Anspruch 13, wobei das Vernetzungsmittel Glutaraldehyd ist und in einer Endkonzentration von wenigstens etwa 0,1% vorhanden ist.

16. Das vernetzte Oxalat-Oxidase-Kristall zur Verwendung nach Anspruch 13, wobei das Vernetzungsmittel Glutaraldehyd ist und in einer Endkonzentration von 4% vorhanden ist.

17. Das vernetzte Oxalat-Oxidase-Kristall zur Verwendung nach Anspruch 13, wobei das Vernetzungsmittel Glutaraldehyd ist und in einer Endkonzentration von 1% vorhanden ist.

18. Das Kristall zur Verwendung nach einem der Ansprüche 13 bis 17, wobei die Oxalat-Oxidase wenigstens 70% der Aktivität der korrespondierenden löslichen Oxalat-Oxidase beibehält.

19. Das Kristall zur Verwendung nach einem der Ansprüche 13 bis 17, wobei die Oxalat-Oxidase wenigstens 95% der Aktivität der korrespondierenden löslichen Oxalat-Oxidase beibehält.

20. Das Kristall zur Verwendung nach einem der Ansprüche 13 bis 17, wobei die Oxalat-Oxidase wenigstens 98% der Aktivität der korrespondierenden löslichen Oxalat-Oxidase beibehält.

21. Eine pharmazeutische Zusammensetzung zur Verwendung bei der Behandlung einer Oxalat-betreffenden Funktionsstörung durch orale Verabreichung umfassend das Kristall nach einem der Ansprüche 13 bis 17.

## Revendications

1. Utilisation de cristaux d'oxalate oxydase dans la fabrication d'un médicament pour le traitement d'un trouble associé avec une concentration d'oxalate élevée chez un mammifère, où les cristaux d'oxalate oxydase sont stabilisés et comprennent de l'oxalate oxydase liée par covalence par un agent réticulant, où le médicament est destiné à l'administration orale.

2. Utilisation selon la revendication 1 où le trouble est choisi dans le groupe consistant en un trouble rénal, un trouble osseux, un trouble hépatique, un trouble gastro-intestinal et un trouble pancréatique.

**3.** Utilisation selon la revendication 1 où le trouble est choisi dans le groupe consistant en l'hyperoxalurie primaire, l'hyperoxalurie entérique, l'hyperoxalurie idiopathique, l'empoisonnement à l'éthylèneglycol, la fibrose kystique, une maladie inflammatoire de l'intestin, l'urolithiase et la néphrolithiase.

**4.** Utilisation selon la revendication 1 où l'agent réticulant est multifonctionnel.

**5.** Utilisation selon la revendication 1 où l'agent réticulant est bifonctionnel.

**6.** Utilisation selon la revendication 5 où l'agent réticulant bifonctionnel est le glutaraldéhyde.

**7.** Utilisation selon la revendication 1 où les cristaux d'oxalate oxydase sont destinés à être administrés sous forme d'une suspension, d'une poudre sèche, d'une capsule ou d'un comprimé.

**8.** Utilisation selon la revendication 1 où l'administration de cristaux d'oxalate oxydase conduit à une réduction de la concentration d'oxalate d'au moins 10 %.

**9.** Utilisation selon la revendication 8 où l'administration de cristaux d'oxalate oxydase conduit à une réduction de la concentration d'oxalate d'au moins 20 %.

**10.** Utilisation selon la revendication 9 où l'administration de cristaux d'oxalate oxydase conduit à une réduction de la concentration d'oxalate d'au moins 30 %.

**11.** Utilisation selon la revendication 8, 9 ou 10 où la réduction est mesurée dans un échantillon biologique choisi dans le groupe consistant en l'urine, le sang, le plasma et le sérum.

**12.** Utilisation selon la revendication 1 où l'oxalate oxydase est produite par recombinaison.

**13.** Cristal d'oxalate oxydase réticulé avec un agent réticulant multifonctionnel destiné à être utilisé dans le traitement d'un trouble lié à l'oxalate par administration orale.

**14.** Cristal d'oxalate oxydase réticulé destiné à être utilisé selon la revendication 13 où l'agent réticulant est le glutaraldéhyde.

**15.** Cristal d'oxalate oxydase réticulé destiné à être utilisé selon la revendication 13 où l'agent réticulant est le glutaraldéhyde et est présent en une concentration finale d'au moins environ 0,1 %.

**16.** Cristal d'oxalate oxydase réticulé destiné à être utilisé selon la revendication 13 où l'agent réticulant est le glutaraldéhyde et est présent en une concentration finale de 4 %.

**17.** Cristal d'oxalate oxydase réticulé destiné à être utilisé selon la revendication 13 où l'agent réticulant est le glutaraldéhyde et est présent en une concentration finale de 1 %.

**18.** Cristal destiné à être utilisé selon l'une quelconque des revendications 13 à 17 où l'oxalate oxydase conserve au moins 70 % de l'activité de l'oxalate oxydase soluble correspondante.

**19.** Cristal destiné à être utilisé selon l'une quelconque des revendications 13 à 17 où l'oxalate oxydase conserve au moins 95 % de l'activité de l'oxalate oxydase soluble correspondante.

**20.** Cristal destiné à être utilisé selon l'une quelconque des revendications 13 à 17 où l'oxalate oxydase conserve au moins 98 % de l'activité de l'oxalate oxydase soluble correspondante.

**21.** Composition pharmaceutique destinée à être utilisée dans le traitement d'un trouble lié à l'oxalate par administration orale comprenant le cristal selon l'une quelconque des revendications 13 à 17.

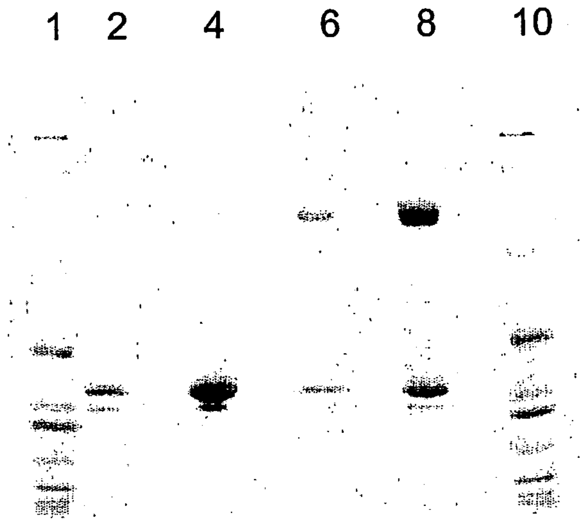


*Figure 1*

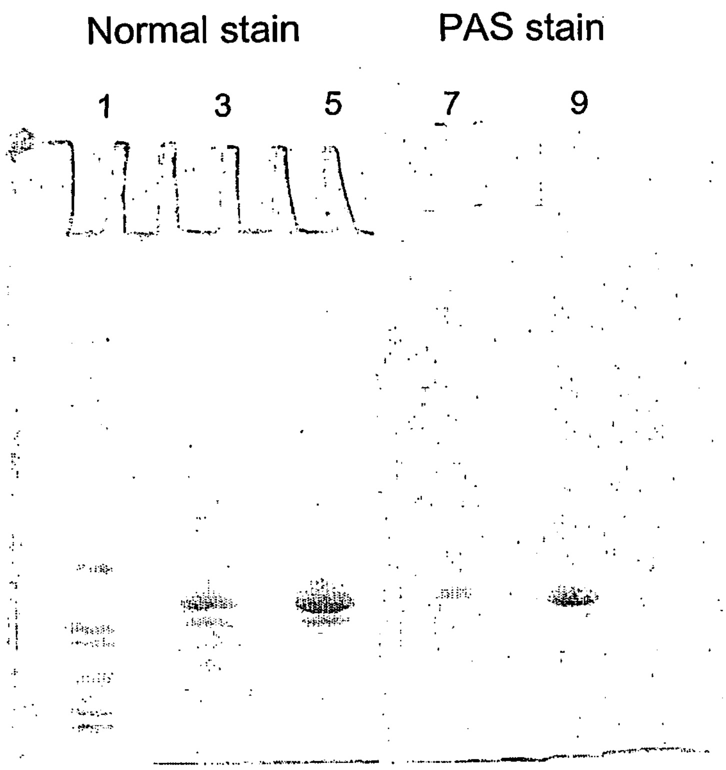


*Figure 2*

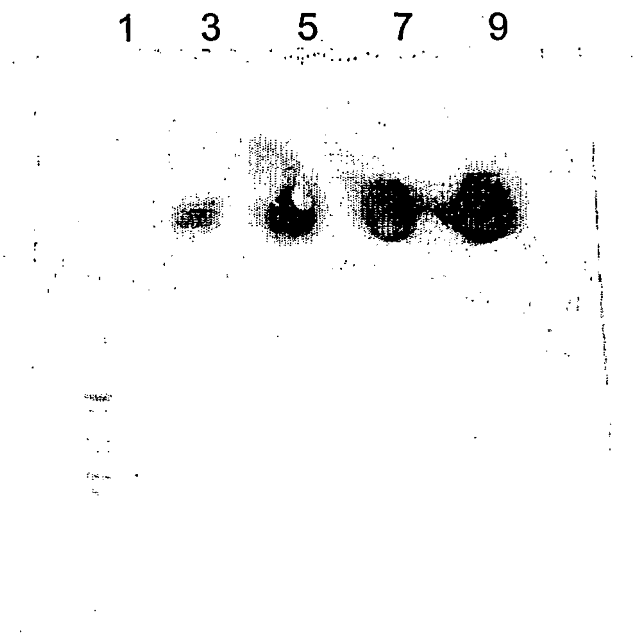


*Figure 3*

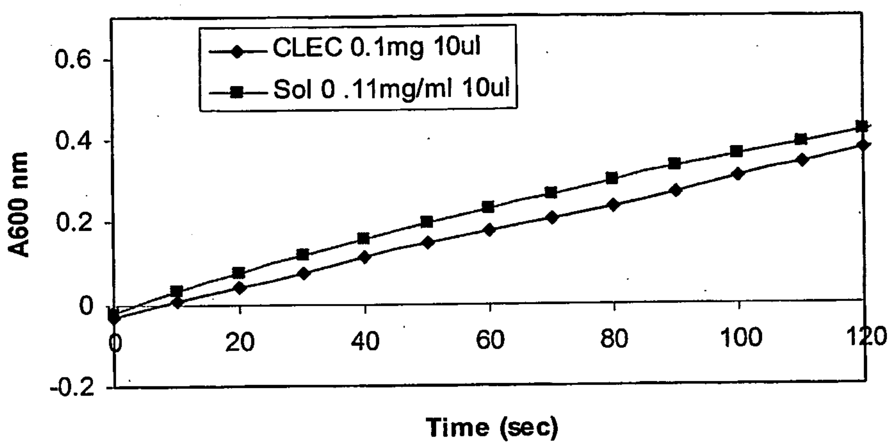
CLEC 0.1mg 10ul
Sol 0 .11mg/ml 10ul
0.6
0.4
0.2
0
-0.2
A600 nm
0
20
40
60
80
100
120
Time (sec)

*Figure 4*

## *Figure 5A*

| | | |
|---|---|---|
| 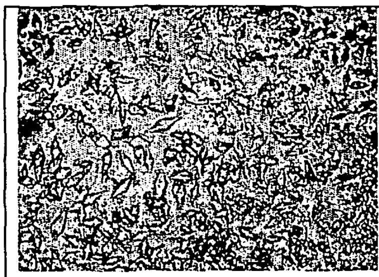 | 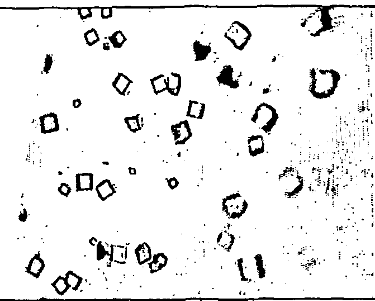 | 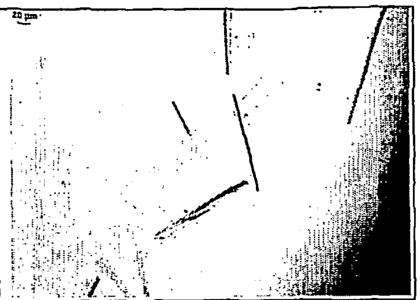 |
| 30% (v/v)PEG 600,<br>0.1M MES,pH6.00<br>5% (w/v) PEG 1000, 10% (v/v) glycerol | 40% (v/v) PEG 400,<br>0.1M Na/K phosphate, pH6.20<br>0.2M NaCl | 40% (v/v) PEG 300,<br>0.1M CHES,pH9.50,<br>0.2M NaCl |
| 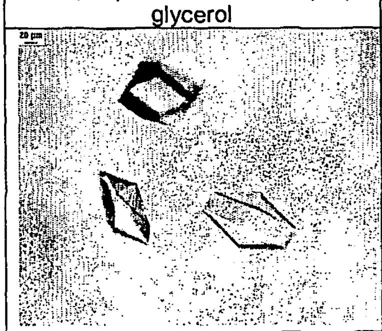 | 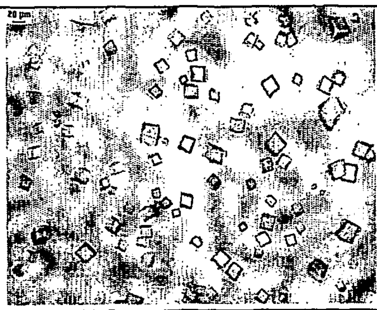 | 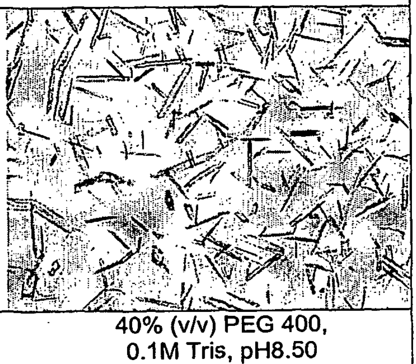 |
| 30% (v/v)PEG 600,<br>0.1M HEPES, pH7.50<br>0.05M Li2SO4, 10% glycerol | 50% (v/v) PEG 200,<br>0.1M Tris, pH7.00<br>0.05M Li2SO4 | 40% (v/v) PEG 400,<br>0.1M Tris, pH8.50<br>0.2M Li2SO4 |
| 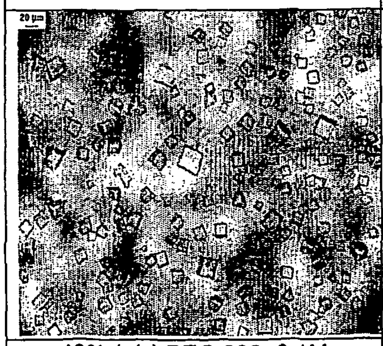 | 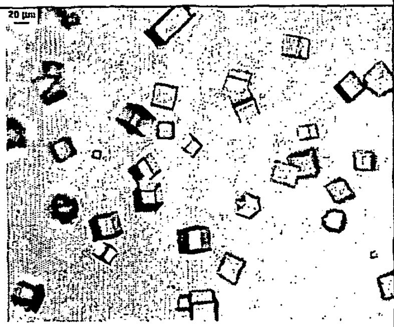 | 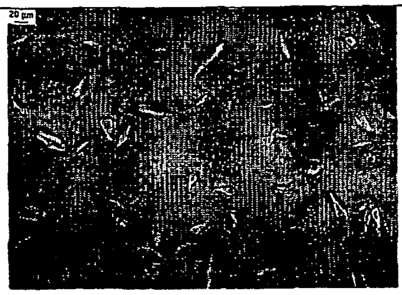 |
| 40% (v/v) PEG 600, 0.1M phosphate-citrate, H4.20 | 40% (v/v) PEG 300,<br>0.1M HEPES, pH7.50<br>0.2M NaCl | 40% (v/v) PEG 400, 0.1M MES, pH6.00<br>5% (w/v) PEG 3000 |

## Figure 5B

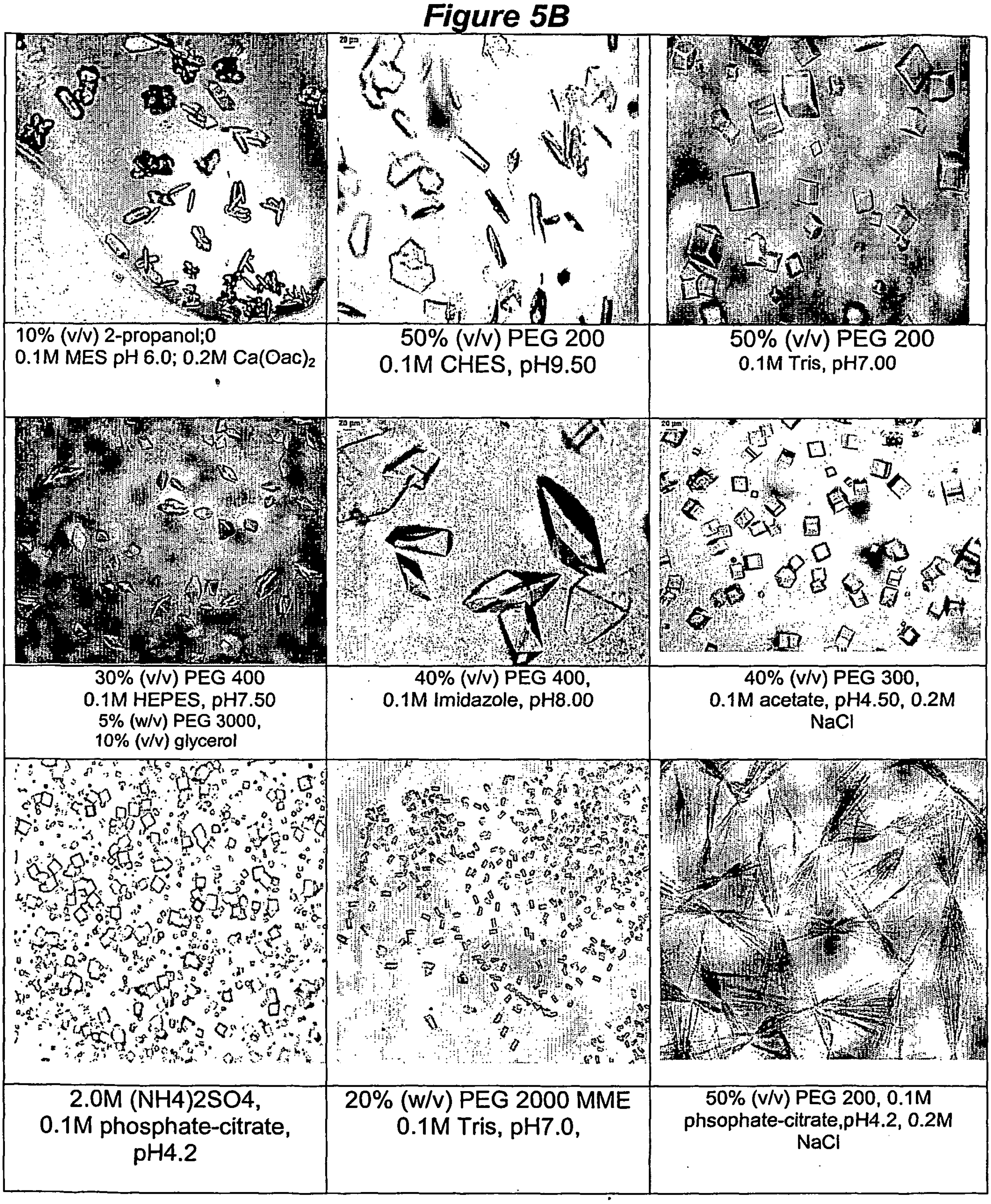
10% (v/v) 2-propanol;0
0.1M MES pH 6.0; 0.2M Ca(Oac)2
50% (v/v) PEG 200
0.1M CHES, pH9.50
50% (v/v) PEG 200
0.1M Tris, pH7.00
30% (v/v) PEG 400
0.1M HEPES, pH7.50
5% (w/v) PEG 3000,
10% (v/v) glycerol
40% (v/v) PEG 400,
0.1M Imidazole, pH8.00
40% (v/v) PEG 300,
0.1M acetate, pH4.50, 0.2M
NaCl
2.0M (NH4)2SO4,
0.1M phosphate-citrate,
pH4.2
20% (w/v) PEG 2000 MME
0.1M Tris, pH7.0,
50% (v/v) PEG 200, 0.1M
phsophate-citrate,pH4.2, 0.2M
NaCl

*Figure 6*

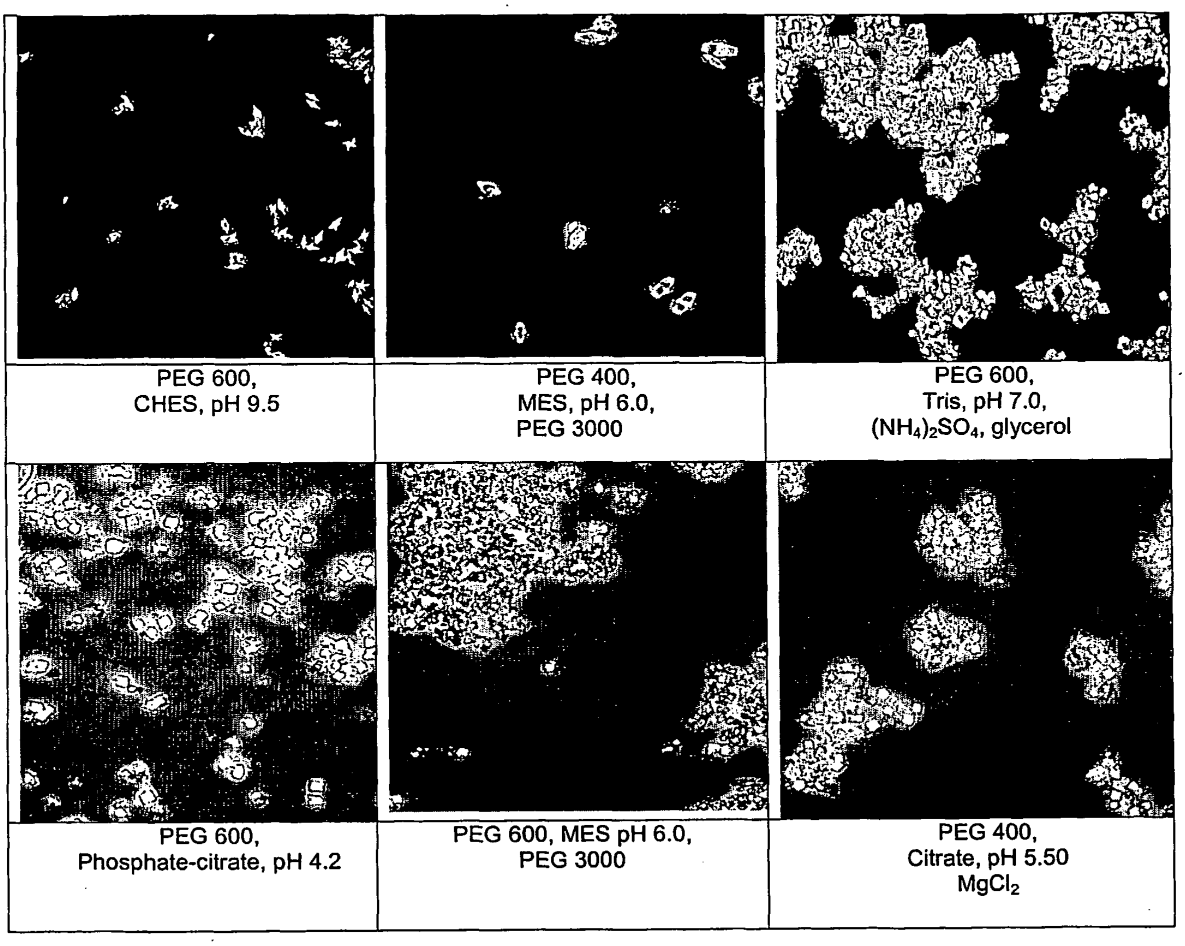
PEG 600,
CHES, pH 9.5
PEG 400,
MES, pH 6.0,
PEG 3000
PEG 600,
Tris, pH 7.0,
$(NH_4)_2SO_4$, glycerol
PEG 600,
Phosphate-citrate, pH 4.2
PEG 600, MES pH 6.0,
PEG 3000
PEG 400,
Citrate, pH 5.50
$MgCl_2$

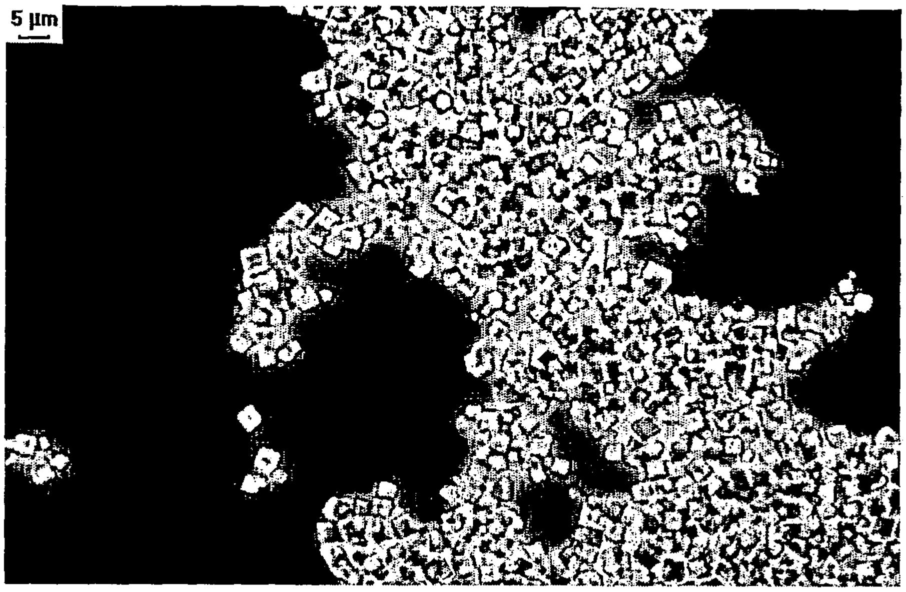


Figure 7A

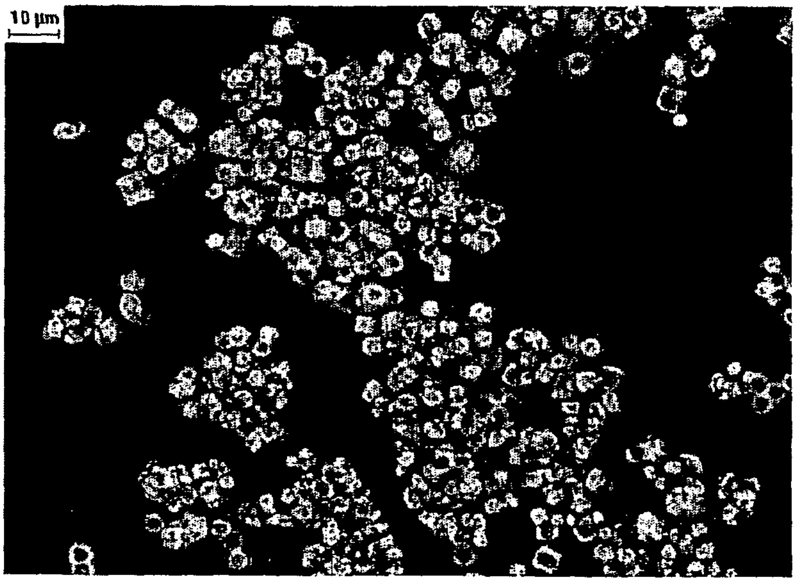


Figure 7B

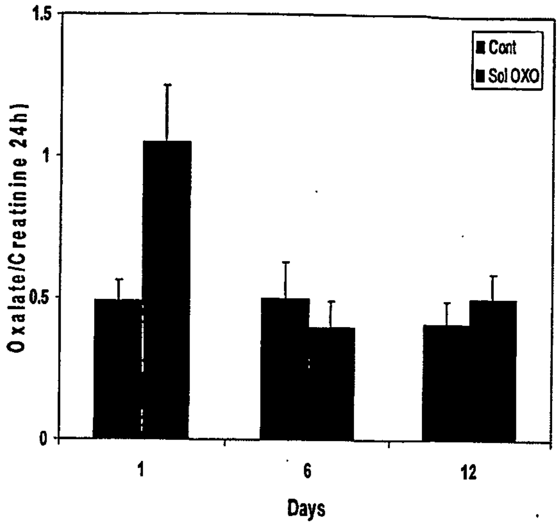


*Figure 8A*

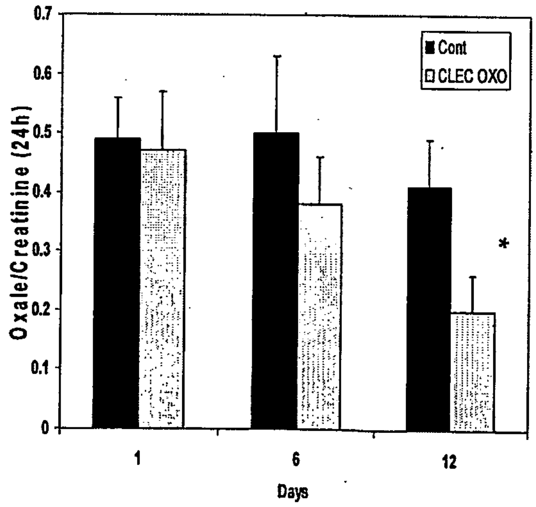


*Figure 8B*

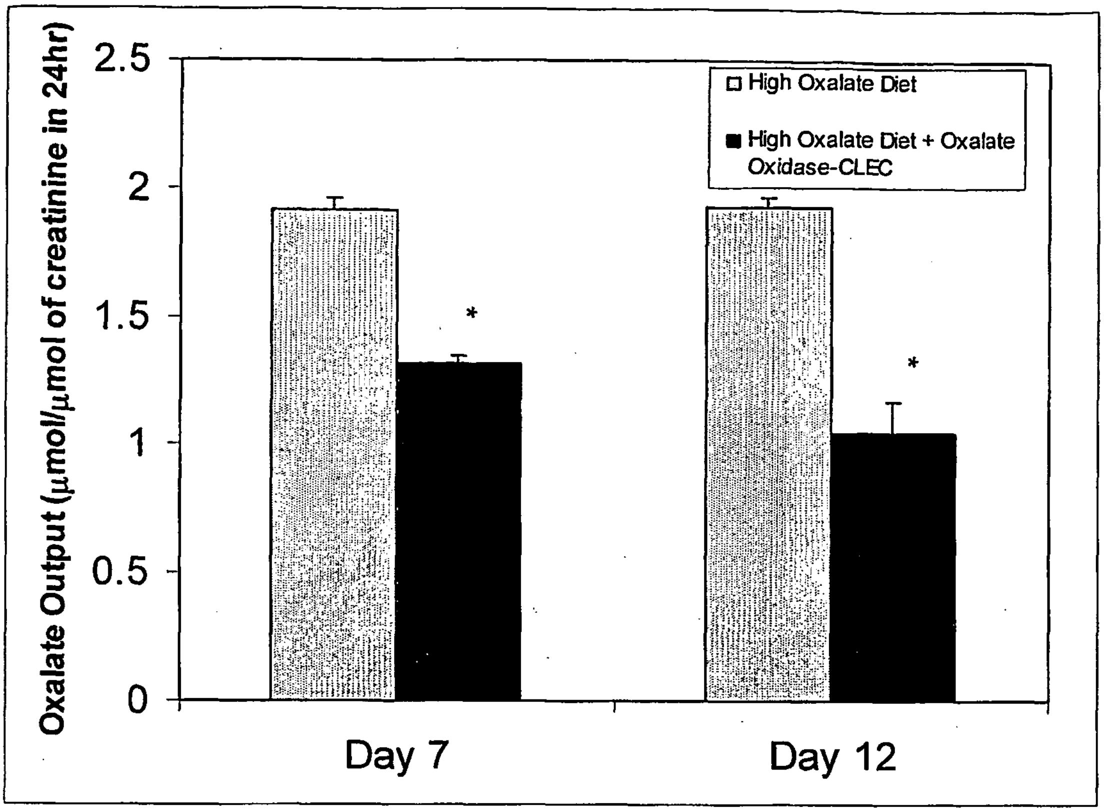
Oxalate Output (μmol/μmol of creatinine in 24hr)
2.5
2
1.5
1
0.5
0
High Oxalate Diet
High Oxalate Diet + Oxalate Oxidase-CLEC
*
*
Day 7
Day 12

*Figure 9*

## *Figure 10*

SEQ ID NO:1:

5'**tcagatccagatccactacaggacttctgtgtcgctgacctagatggaaag**
**gctgtctcagtgaacggacatacttgtaagccaatgtctgaggcaggagatga**
**cttcctcttctcgtctaagctgactaaggcaggaaacacgtctactcctaacg**
**gttcagcagttacggaactagacgtagctgaatggccaggtactaacactctg**
**ggtgtgtcaatgaaccgtgttgacttcgctccaggaggtaccaacccacctca**
**catccatccacgtgcaactgagatcggtatggtgatgaaggtgaactccttg**
**ttggaatcctcggtagccttgactcaggaaacaagctctactccagagtagtg**
**cgtgctggagaaactttcgtcatcccacgtggactcatgcacttccagttcaa**
**cgttggtaagacggaagcatacatggttgtgtccttcaacagccagaaccctg**
**gtatcgtcttcgtgccactcacactcttcggttccgatcctcctatcccaacg**
**ccagtgcttaccaaggctctccgagttgaggctggagtcgtggaacttctcaa**
**gtccaagttcgcaggtggttcttaa** 3' [SEQ ID NO:1]

SEQ ID NO:2

SDPDPLQDFCVADLDGKAVSVNGHTCKPMSEAGDDFLFSSKLTKAGNTSTPNGSA
VTELDVAEWPGTNTLGVSMNRVDFAPGGTNPPHIHPRATEIGMVMKGELLVGILGS
LDSGNKLYSRVVRAGETFVIPRGLMHFQFNVGKTEAYMVVSFNSQNPGIVFVPLTL
FGSDPPIPTPVLTKALRVEAGVVELLKSKFAGGS [SEQ ID NO:2]

SEQ ID NO:3

Native barley oxalate oxidase gene sequence from GenBank
(ACCESSION L15737; VERSION L15737.1 GI:289356)

```
1   tccgacccag acccactcca ggacttctgc gtcgcggacc tcgatggcaa ggcggtctcg
61  gtgaacgggc atacgtgtaa gcccatgtcg gaggccggcg acgacttcct cttctcgtcc
121 aagctgacca aggccggcaa cacgtccacc ccgaacggct cggccgtgac ggagctcgac
181 gtggccgagt ggcccggtac gaacacgctg ggcgtgtcca tgaaccgtgt ggacttcgcg
241 ccgggggcca ccaacccgcc gcacatccac ccgcgtgcaa ccgagatcgg catggtgatg
301 aaaggtgagc tcctcgttgg aatcctcggc agccttgact ccggaaacaa gctctactcc
361 agggtggtgc gtgccggaga gactttcgtc atcccgcgcg gcctcatgca cttccagttc
421 aacgttggta agacggaagc ctacatggtt gtgtccttca acagccagaa ccctggcatc
481 gtcttcgtgc cgctcacact cttcggctcc gaccctccca tccccacgcc cgtgctcacc
541 aaggctctcc gggtggaggc cggagtcgtg gaacttctca agtccaagtt cgccggtggg
601 tct
```

*Figure 11*

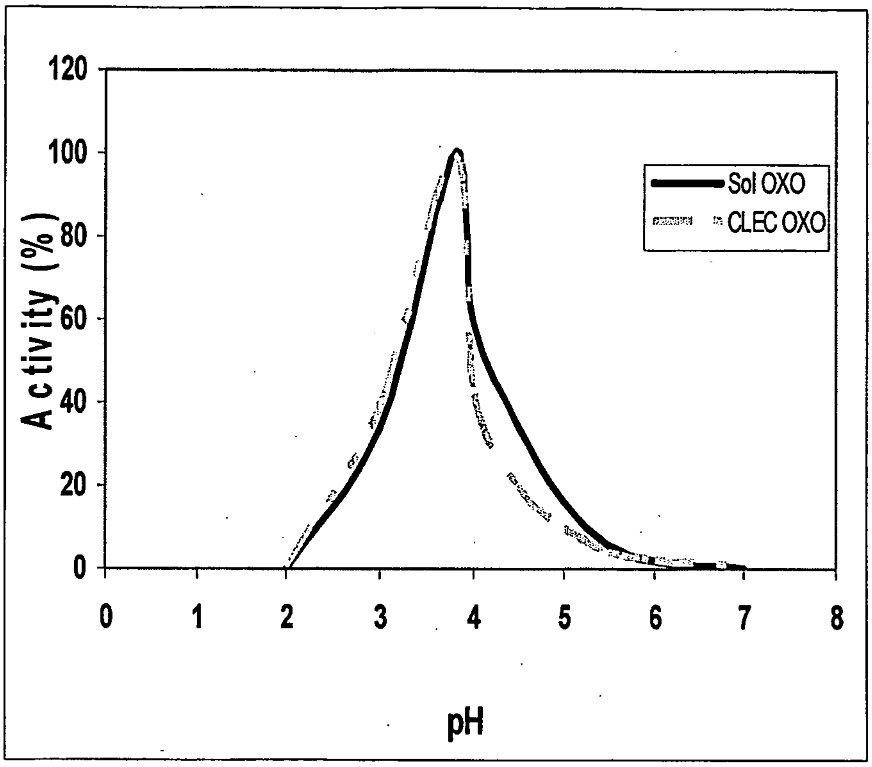

*Figure 12*

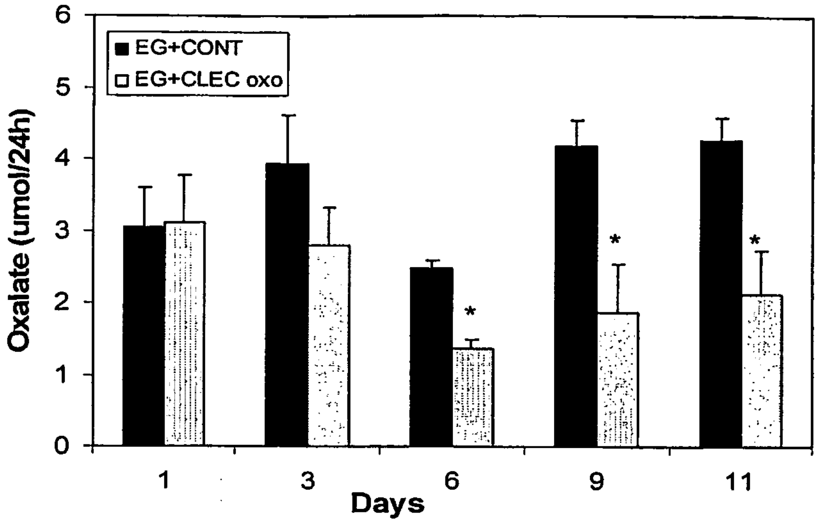

*Figure 13*

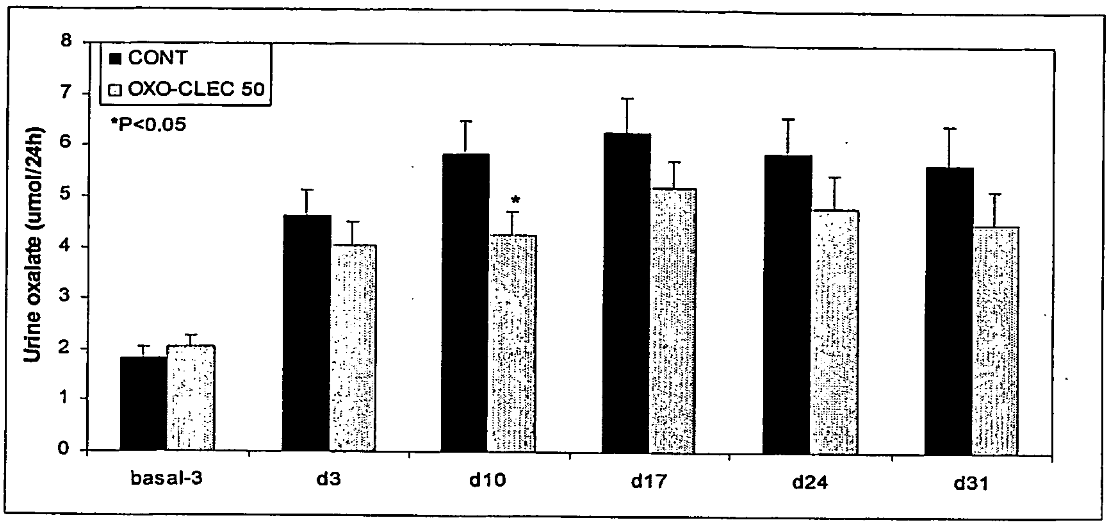

Urine oxalate (umol/24h)
8
7
6
5
4
3
2
1
0
CONT
OXO-CLEC 50
*P<0.05
*
basal-3
d3
d10
d17
d24
d31

*Figure 14*

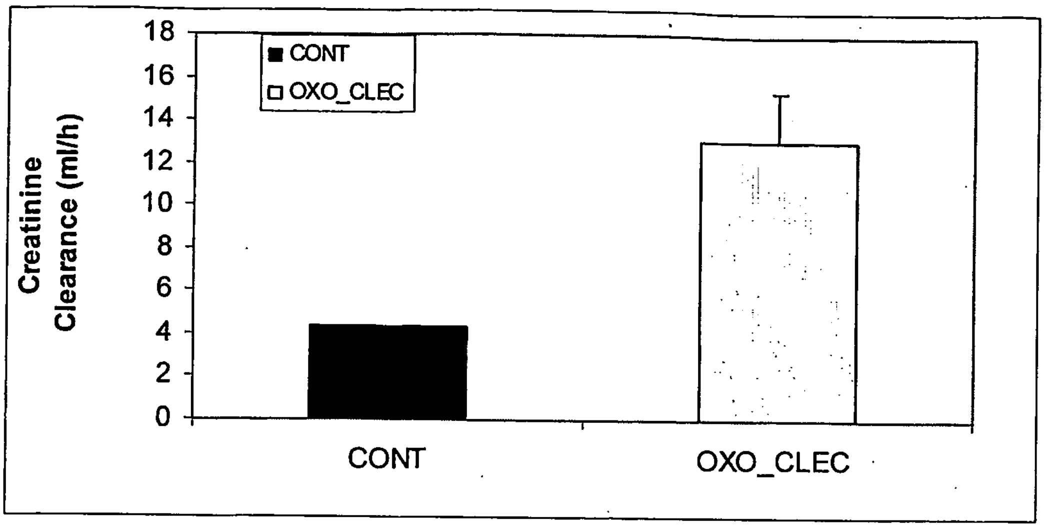
Creatinine Clearance (ml/h)
18
16
14
12
10
8
6
4
2
0
CONT
OXO_CLEC
CONT
OXO_CLEC

*Figure 15*

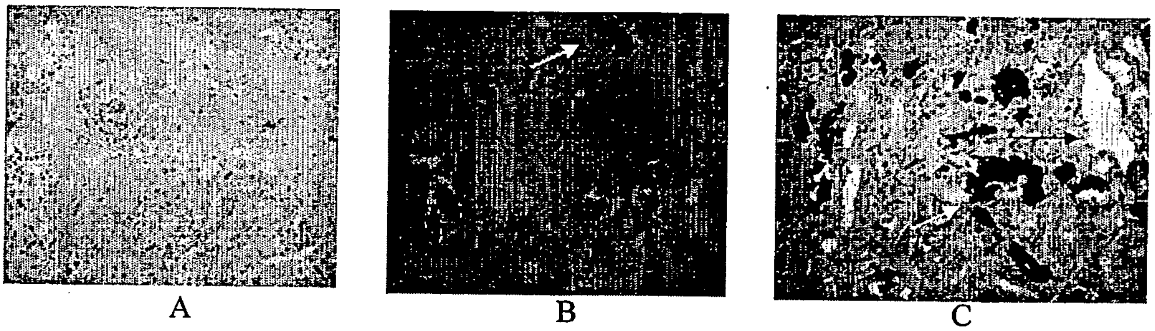

A B C

*Figure 16*

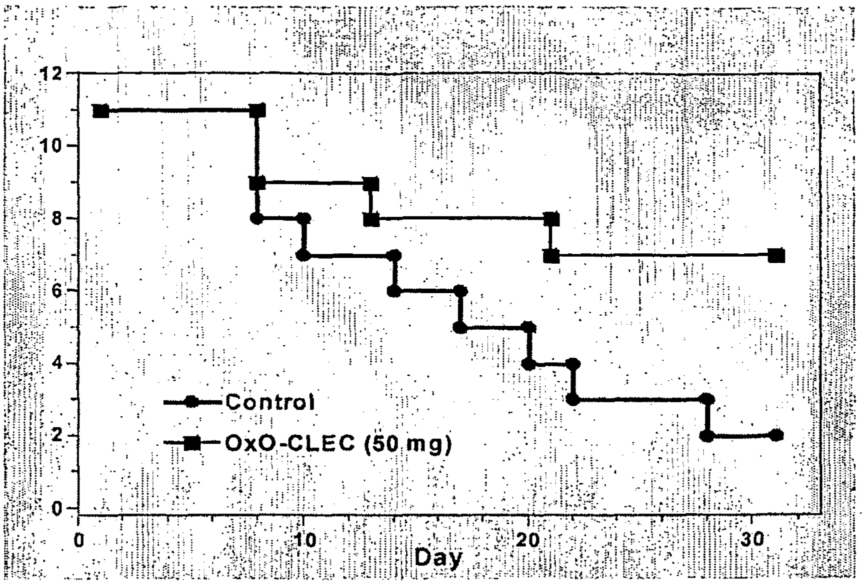

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6200562 B
- US 6355242 B
- US 6699469 B
- US 20040234514 A
- US 5976529 A
- US 6140475 A
- US 6541606 B


### Non-patent literature cited in the description


- **LEUMANN et al.** *Nephrol. Dial. Transplant.,* 1999, vol. 14, 2556-2558
- **EARNEST.** *Adv. Internal Medicine,* 1979, vol. 24, 407-427
- **LEUMANN et al.** *J. Am. Soc. Nephrol.,* 2001, vol. 12, 1986-1993
- **MONICO et al.** *Kidney International,* 2002, vol. 62, 392-400
- **BARRET.** Structure of Methals. McGraw-Hill, 1952
- **LIU et al.** *Zhi Wu Sheng Li Yu Fen Zi Sheng Wu Xue Xue Bao,* 2004, vol. 30, 393-8
- **RODRIGUIEZ-LOPEZ et al.** *FEBS Lett.,* 2001, vol. 9, 44-48
- **PUNDIR et al.** *Chin. J. Biotechnol.,* 1999, vol. 15, 129-138
- **AGUILAR et al.** *Arch. Biochem. Biophys.,* 1999, vol. 366, 275-82
- **ALTSCHUL et al.** *J. Mol. Biol.,* 1990, vol. 215, 403-410
- **NEEDLEMAN et al.** *J. Mol. Biol.,* 1970, vol. 48, 444-453
- **MEYERS et al.** *Comput. Appl. Biosci.,* 1988, vol. 4, 11-17
- **WOO et al.** *FEBS Letters,* 1998, vol. 437, 87-90
- **WOO et al.** *Nature Struct. Biol.,* 2000, vol. 7, 1036-1040
- **MCPHERSON et al.** *Methods Enzymol.,* 1985, vol. 114, 112-20
- **GILLILAND.** *J. Crystal Growth,* 1998, vol. 90, 51-59
- Pierce Catalog and Handbook. Pierce Chemical Company, 1994
- **S. S. WONG.** Chemistry of Protein Conjugation and Cross-Linking. CRC Press, 1991
- **T. W. GREEN.** Protective Groups in Organic Synthesis. John Wiley & Sons, 1981
- *Angewante Chemise Inl. Ed. Engl.,* 1996, vol. 35, 2056
- Pierce Catalog and Handbook. 1994
- Physician's Desk Reference. Medical Economics Company, 2002
- Remington: The Science and Practice of Pharmacy. Lippincott, Williams & Wilkins, 2000
- **WHITTAKER et al.** *J. Biol. Inorg. Chem.,* 2002, vol. 7, 136-145